# EUROPEAN PATENT APPLICATION

(11) **EP 1 347 042 A1**
(43) Date of publication of application: **24.09.2003**
(21) Application number: 01998625.6
(22) Date of filing: 30.11.2001
(51) Int. Cl.: C12N 7/01, C12N 15/19, C12N 15/33, A61K 48/00

(54) **A VIRUS WHICH CAN EXPRESS TUMOR ANGIOSTATIN FACTOR WITH HIGH EFFICIENCY IN SPECIFIC TUMOR CELLS AND THE USE OF IT**

(30) Priority: 01.12.2000 CN 00127680
(71) Applicant: Virgene Biotechnology Limited, Hong Kong (CN)
(72) Inventor: QIAN, Qijun, Shanghai 200438 (CN); CHE, Xiaoyan, Shanghai 200438 (CN); SHAM, Shuntong, Shanghai 200438 (CN); WU, Mengchao, Shanghai 200438 (CN)
(74) Representative: Geismar, Thierry
(86) International application number: CN0101596
(87) International publication number: WO02044347

(57) **Abstract**

The invention disclosed a virus, which can express tumor angiostatin factor with high efficiency and proliferate in specific tumor cell and the use of it. Said virus mainly propagates in tumor cells and it can specifically kill the tumor cells straightforwardly. By adding the nucleotide sequences encoding tumor angiostatin factor in the non-proliferating necessary region of the viral genome. With the replication of the virus in the tumor cells, the nucleotide sequences encoding the tumor angiostatin factor multiple. So it can express tumor angiostatin factor with efficiency in specific tumor cell and restrain the forming of the tumor blood vessel, and control the tumor forming, developing and tranferring.

## Description

### Field of the Invention

The present invention relates to the field of life science, particularly, relates to a virus specifically propagating in tumor cells and capable of expressing an angiogenesis inhibitor at high level, and to uses thereof.

Malignant tumors badly endanger the life and health of human beings. Presently, the routine therapy for malignant tumors is still operation, radiotherapy and chemotherapy. This kind of routine therapy cannot achieve desirable effects for most tumors, generally concomitant with obvious toxic effects, including bone marrow depression that endangers life. In the last few years, great advances have been made on the study of the nascent blood vessel of tumors. Folkman's research group proposed the "the on-off mechanism of angiogenesis" during the initiation and development of tumors, revealling the molecular mechanism of the formation of microvessel of tumors. When the tumor's radius is less than 2 mm, its existence mainly depends on the nutritious substances and oxygen diffused in the surroundings of cells. Along with the enlargement of tumor size, the tumor or host tissues will create nascent vascular net to supply the neoplasm body with nourishments. The formation of nascent vascular net depends on the results of the interaction between factors inducing and repressing the formation of nascent vascular net in the microenvironment surrounding the tumors. During the initiation of tumors and the formation of the metastasis focuses, the factors inducing and promoting the formation of renascent vascular net are predominant, such as, fibroblast growth factor( bFGF), vessel endothelial cell growth factor(VEGF), transforming growth factor-α ( TGF-α ), tumor necrosis factor-α ( TNF-a), blood platelet-derived endothelial growth factor(PDEGF), interleukin-8(IL-8), plasminogen activator(PA) and matrix metalloproteinase ( MMP), while the factors repressing the formation of nascent vessels, such as endostatin, angiostatin, interferon-α , interferon -β , interferon-γ, thrombospondin, platelet factor 4 gene, plasminogen activator inhibitor( PAD gene and the fibronectingene, are in an inferior position, thus leading to the formation of the nascent vesseles of tumors. The nascent vesseles transport nourishments for the rapid propagation of tumor cells and also make the long-distance metastasis of tumors come true. Repressing the formation of nascent vessels of tumors can block the nutrion supply of tumors, whereby resulting in the death of tumor cells because of malnutrition, and the obvious depauperation of tumors, further complete elimination. Meanwhile, repressing the formation of nascent vessels of tumors can also block the passages for tumor metastasis.

Presenetly there are many kinds of inhibitive factors blocking the formation of the nascent vessels of tumors, which have entered the clinical trial. But due to the shorter half time of inhibitive factors of nascent angiogenesis, the treatment is required everyday. At the same time, the required amount is usually much more, for example, the therapeutic dosage of endostatin and angiostat is 10-20 mg/ Kg/day. It is difficult for conventional bioengineering techniques to realize it.

It is well known that, for a long time, there are a lower efficiency of gene transfection and a lower expression amount of antioncogene in methods of the tumor gene therapy. However, treatments based on viruses specifically propagating in tumor cells utilize the property that this kind of viruses specifically replicate and propagate only in tumor cells, lyse the tumor cells and release the virons along with the replication and propagation of viruses in tumor cells. Then the released virons infect, propagate and lyse other tumor cells again. Thus an amplification effect is produced; thereby viruses can diffuse in all tissues and organs of the human body. They can infect all tumor cells, kill local and metastatic tumors. Basically, viruses specifically propagating in tumors cannot propagate in normal cells; they therefore do not affect normal cells surrounding tumor cells. Presently, the ONYX Drug Company of the United States tried to utilize the Elb 55 kDa protein-deficient virus ( ONYX-015) alone for treating tumors, but its clinical effectual ratio is only 15-20%( Nemunaitis, J. et al., *Cancer Res*.,2000,60:(22) 6359; US 5677178; US 5801029).

The present inventor combines creatively the therapy of suppressing the blood vessel of tumors with the therapy based on viruses specifically propagating in tumors. It is the first time to present the therapy of suppressing blood vessel-virus treatment, i.e., utilizing viruses specifically propagating in tumors to carry an angiogenesis inhibitor gene and successfully achieving the purpose of specific replication and propagation of viruses in tumor cells and production of high concentration of viruses, thereby destroying tumor cells directly. Meanwhile, along with the replication of virus in tumor cells, the carried angiogenesis inhibitor gene was massively expressed to produce synergistic effects. The therapy of suppressing blood vessel-virus treatment described in the present invention overcame the difficult problem of produing angiogenesis inhibitor massively and meanwhile, the effectual ratio increased obviously compared to that of the methods using viruses specifically propagating in tumors for treating only.

### Summary of the Invention

One objective of the present invention is to provide a virus specifically propagating in tumor cells and capable of expressing angiogenesis inhibitor at high level, wherein the non-essential region of genome of the said virus comprises a nucleotide sequence encoding the angiogenesis inhibitor. The said virus is selected from the group consisting of:
1) a wild-type virus specifically propagating in tumor cells;
2) a recombinant virus containing a cis-acting element specifically activated in tumor cells between the transcriptional start site and the encoding start site in essential genes for propagation of virus;
3) a recombinant virus comprising at least one loss of protein function in essential region for propagation and capable of specifically propagating in tumor cells.

Another objective of the present invention is to provide a method using the viruses described in the present invention for treating mammal tumors, especially human tumors. It includes the steps as follows: 1) *in vitro* or *in vivo* infecting tumor cells using the said virus, 2) making the virus selectively replicate and propagate substantially limited in tumor cells, leading to the increase of the copy numbers of the nucleotide sequence encoding the angiogenesis inhibitor and the expression amount of angiogenesis inhibitor in the tumor cells, whereby repressing the vascularization of tumors, specifically killing tumor cells directly to repress the formation, growth and metastasis of tumors. Properly, the methods described in the present invention further include administration of the chemical antineoplastic drugs prior to, concurrently with and/or subsequent to the infection of tumor cells with viruses described in the present invention.

Still another objective of the present invention is to provide uses of the viruses described in the present invention for inhibition of the growth of tumor cells.

### Detailed Description of the Invention

The present invention provides a virus specifically propagating in tumor cells and capable of expressing an angiogenesis inhibitor at high level. The nucleotide sequence encoding an angiogenesis inhibitor is inserted into the non-essential region of genome of a virus specifically propagating in tumor cells and the virus selectively propagates in tumor cells, but substantially does not propagate in normal cells. Along with the replication and propagation of the virus in the tumor cells, the copy numbers of the nucleotide sequence encoding the angiogenesis inhibitor are increased; thereby the expression amount of angiogenesis inhibitor is increased. The said virus is selected from the group consisting of:
1) a wild-type virus specifically propagating in tumor cells;
2) a recombinant virus containing a cis-acting element specifically activated in tumor cells between the transcriptional start site and the encoding start site in essential region for propagation of the virus;
3) a recombinant virus containing at least one kind of losses of protein function in essential region for propagation and capable of specifically propagating in tumor cells.

Many kinds of wild-type viruses may be selected and used in the present invention, as long as they can specifically propagate in one or more kinds of tumor cells. It is preferable to use an adenovirus and a herpes simplex virus.

The essential region for propagation of virus described in the present invention may vary with the various viruses used, which is readily known to those skilled in the art. The viruses of the present invention can be a recombinant virus which contains at least one cis-acting element specifically activated in tumor cells between the transcriptional start site and the encoding start site in essential region for propagation of the virus, wherein the said cis-acting element is selected from the group consisting of: the enhancer and promoter of a -fetal protein, the enhancer and promoter of carcinoembryonic antigen, the enhancer and promoter of tyrosinase, the enhancer and promoter of ErbB2, the enhancer and promoter of ErbB3, the enhancer and promoter of ErbB4, the enhancer of DF3 mammary cancer-related antigen, the enhancer and promoter of prostaglandin-specific antigen, the enhancer and promoter of glandular kallikrein, Orip in EB virus, FR enhancer in Orip of EB virus, BamHI C-promoter of EB virus, Orip in EB virus combined with BamHI C-promoter of EB virus, FR enhancer in Orip of EB virus combined with the basic promoter of thymidine kinase of herpes simplex virus or the basic promoter of SV40 and the cis-acting elements specifically activated in the cells infected or latently infected by EB virus.

When the said virus used therein is an adenovirus, the essential gene for propagation of the virus is a virus early gene, and the said essential gene for propagation of the virus is one of the following early expression genes of an adenovirus: E1A, E1B, E2 or E4.

The viruses described in the present invention further may be a recombinant virus containing at least one kind of losses of protein function in essential region for propagation and capable of specifically propagating in tumor cells, wherein the said losses of protein function may be achieved by point mutation, deletion and insertion mutation of a gene, thereby resulting in the said losses of protein function. In one embodiment of the present invention, the said virus is a recombinant adenovirus, wherein the E1B55Kda gene of this adenovirus was mutated by point mutation, deletion mutation and insertion mutation, resulting in the protein function abnormality of EIB55Kda. In one embodiment of the present invention, the said virus is a recombinant adenovirus, wherein the E1B 19kDa gene of this adenovirus was mutated by point mutation, deletion mutation and insertion mutation, resulting in the protein function abnormality of E1B 19kDa. In one embodiment of the present invention, the said virus is a recombinant adenovirus, wherein the E1A gene of this adenovirus was mutated by point mutation, deletion mutation and insertion mutation, resulting in the protein function abnormality of E1A.

In one embodiment of the present invention, the said virus is a recombinant herpes simplex virus, wherein ICP6 gene of this adenovirus was mutated by point mutation, deletion mutation and insertion mutation, resulting in the protein function abnormality of ICP6. In one embodiment of the present invention, the said virus is a recombinant herpes simplex virus, wherein the double-copied ICP34.5 gene of this adenovirus was mutated by point mutation, deletion mutation and insertion mutation, resulting in the protein function abnormality of the double-copied ICP34.5.

In the said viruses of the present invention, the nucleotide sequences encoding angiogenesis inhibitor include, but are not limited to, the sequences selected from the group consisting of those encoding: endostatin, angiostatin( Kringle1-4 structure in plasma plasminogen), Kringle1-5 structure in plasma plasminogen, Kringlel-3 structure in plasma plasminogen, Kringle1-3 structure in plasma plasminogen plus Kringle5 structure, Kringlel structure in plasma plasminogen, Kringle2 structure in plasma plasminogen, Kringle3 structure in plasma plasminogen, Kringle5 structure in plasma plasminogen, interferon-α, interferon-β , interferon-γ, thrombospondin I, platelet factor 4, plasminogen activator inhibitor and fibronectin. It is generally known to those of skills in the art that proteins, as long as they have the function as angiogenesis inhibitor, can all be used in the present invention.

To enhance the expression efficiency of the said nucleotide sequence encoding the angiogenesis inhibitor, it is preferable to use a nucleotide sequence coding for secretive signal peptide in the said sequence. In one embodiment of the present invention, nucleotide sequences for the said secretory signal peptides include, but are not limited to, the above-mentioned signal peptide of angiogenesis inhibitor itself, the signal peptide of M-oncostatin, the signal peptide of immunoglobulin K chain. Any of signal peptides capable of secreting efficiently angiogenesis inhibitors described in the present invention, either homologous or heterologous, can all be used in the present invention.

In addition, nucleotide sequences encoding the said angiogenesis inhibitors in the viruses described in the present invention are still preferably controlled under promoters. The said promoters include, but are not limited to, SV40 promoter, RSVLTR promoter and/or IE promoter of human cytomegalovirus. Any of promoters capable of directing efficiently expression of angiogenesis inhibitors described in the present invention, either homologous or heterologous, can all be used in the present invention.

In the present invention, the construction of tumor-specific viruses, resulting in the viruses specifically propagating and replicating in the tumor cells, but not propagating and replicating in the normal cells, is primarily achieved by the following methods:

(1) selectively control of essential genes for propagating of an virus: The regulation of activation of gene transcription is affected by interaction between trans-acting factors (e.g., transcription factors) and cis-acting elements. The absence or presence of some transcriptional factors may affect the level of gene transcription. The present invention utilizes cis-acting elements (including promoters or enhancers) specifically activatied in viral tissues to control target genes and make the target genes express specifically in tumor cells, but do not express or express at lower level in normal cells. The essential genes for propagation and replication of virus are controlled by tumor tissue-specific promoters and enhancers, resulting in the expression of the genes for propagation and replication of the virus only in tumor cells; therefore the virus propagates only in tumor cells, but maily not in normal cells.

Cell specific response elements used in the present invention are composed of cis-acting elements activated specifically in tumor cells. The cis-elements may be any one of the following: the enhancer and promoter of α -fetal protein (AFP), which are the enhancer and promoter activated in liver cancer cells; the enhancer and promoter of carcinoembryonic antigen (CEA), which are the enhancer and promoter activated in stomach cancer cells and colon cancer cell; the enhancer and promoter of tyrosinase, which are the enhancer and promoter activated in melanoma cells; the enhancer and promoter of ErbB2, which are the enhancer and promoter activated in mammary cancer cells; the enhancer and promoter of ErbB3, which are the enhancer and promoter activated in mammary cancer cells; the enhancer and promoter of ErbB4, which are the enhancer and promoter activated in mammary cancer cells and stomach cancer cells; the enhancer of DF3 mammary cancer-related antigen (MUC1), which are the enhancer and promoter activated in mammary cancer cells; the enhancer and promoter of prostaglandin-specific antigen, wherein the enhancer of prostaglandin-specific antigen locates in nt-5322--nt-3739 to the transcriptional start site of the prostaglandin-specific antigen, the promoter is located at nt-540-- nt+12 to the transcriptional start site of the prostaglandin-specific antigen and the enhancer and promoter can be specifically activated in prostatic cells and prostatic cancer cells; the enhancer and promoter of glandular kallikrein, which are activated specifically in prostatic cells and prostatic cancer cells; Orip in Epstcin―Barr virus ( generally abbreviated to EB virus), FR in Orip of EB virus, BamHI C-promoter of EB virus, Orip in EB virus combined with BamHI C-promoter of EB virus, FR in Orip of EB virus combined with the basic promoter of thymidine kinase of herpes simplex virus or the basic promoter of SV40 and the cis-acting elements specifically activated in the cells infected or latently infected by EB virus.

The present invention provides a propagative recombinant virus specifically killing tumor cells. The virus comprises at least one cis-acting element from the essential genes for propagation of virus activated in tumor cells, wherein the said cis-acting element was inserted into the region between the transcriptional start site and the encoding start site of gene in the essential gene for propagation of virus. The said cis-acting element is specifically activated in tumor cells and produces transcription activity, but not activated in normal cells and does not produce transcription activity The cis-acting element may be one of the following sequences: the enhancer and promoter of α -fetal protein (AFP), the enhancer and promoter of carcinoembryonic antigen (CEA), the enhancer and promoter of tyrosinase, the enhancer and promoter of ErbB2, the enhancer and promoter of ErbB3, the enhancer and promoter of ErbB4, the enhancer of DF3 mammary cancer-related antigen (MUC1), the enhancer and promoter of prostaglandin-specific antigen, the enhancer and promoter of glandular kallikrein, Orip in EB virus, FR enhancer in Orip of EB virus and BamHI C-promoter of EB virus, Orip in EB virus combined with BamHI C-promoter of EB virus, FR enhancer in Orip of EB virus combined with the basic promoter of thymidine kinase of herpes simplex virus or the basic promoter of SV40 and the cis-acting elements specifically activated in the cells infected or latently infected by EB virus. The above-mentioned essential genes for propagation of virus are early expression genes of virus. In one embodiment of the present invention, herpes simplex virus is used and its essential genes for propagation of virus include the early expression gene ICP4. In another embodiment of the present invention, the above-mentioned virus may be an adenovirus. Its essential genes for propagation of virus include at lease one of the following early expression genes of adenovirus: E1A, E1B, E2 and E4.

(2). selectively depleting the protein functions encoded by the the genes which are essential for replication of virus in normal cells but not essential in tumor cells:

There are some differences in the expression of genes between normal cells and tumors. Some essential genes for replication of virus in the normal cells are not needed in tumor cells. Therefore, it is expected that deletion of the protein functions encoded by these genes can result in replication of virus specifically in tumor cells but not in normal cells.

The loss of the protein functions of virus may be anyone of the following: E1B 55kDa gene of adenovirus was mutated by point mutation, deletion mutation and insertion mutation, resulting in the protein function abnormality of EIB55kDa. E1B 19kDa gene of adenovirus was mutated by point mutation, deletion mutation and insertion mutation, resulting in the protein function abnormality of E1B 19kDa. E1A gene of adenovirus was mutated by point mutation, deletion mutation and insertion mutation, resulting in the protein function abnormality of E1A. ICP6 gene of herpes simplex virus was mutated by point mutation, deletion mutation and insertion mutation, resulting in the protein function abnormality of ICP6. ICP34.5 gene of herpes simplex virus was mutated by point mutation, deletion mutation and insertion mutation, resulting in the protein function abnormality of ICP34.5.

(3). replication of virus depending on the abnormality of a certain signal transduction passway:

After Reovirus infection, the transcription of its early viral genes can activate the double-RNA dependent protein kinase (PKR) and the kinase can repress the transcription of other genes of the virus; therefore the virus can not replicate efficiently. However, when Ras is in activated status in the cell, it can repress the kinase, resulting in the active replication of the virus. Ras gene is an oncogene, and when it is activated abnormally, carcinomatous change will occur. The replication and propagation of Reovirus are dependent on the signal passway of Ras activated abnormally, i.e., the virus replicates and propagates in tumor cells expressing Ras at high level.

(4). selectively entering tumor cells:

Viruses can bind some particular tumor tissues by changing their surface binding proteins, and therefore viruses only infect the particular tumor tissues. Presently, the reform in this aspect mainly concentrates on the coat protein-fibrin (Fiber) of adenovirus, penton and hexon proteins; especially in the head HI ring of fibrin or the C terminus, it is most frequently, including some kind of high affinity ligands, short peptides and the Fab region of antibody inserted into the membrane surface of tumors.

The present invention provides a virus specifically propagating in tumor cells and capable of expressing an angiogenesis inhibitor at high level and its nucleotide sequence encoding the angiogenesis inhibitor is the nucleotide sequence encoding the one containing endostatin.

Endostatin is a 20 Kda C-terminus fragment of a collagen XVIII and it can specifically repress the propagation of endothelial cells of blood vessels without affecting other cells. The experiments carried out by Folkman et al. demonstrated that endostatin had a stronge repressive activity against angiogenesis induced by tumors, thereby repressing the formation and metastasis of tumors. Furthermore, there was no drug-resistance produced upon repeated administration and also no obvious toxicity even several hundred folds of treatment dosage was used. Since the endostatin is the C-terminus fragment of collagen XVIII, it doesn't have the secretory signal peptide. For gene therapy, it is required to add a secretory signal peptide. The signal peptide of M-oncostatin and the signal peptide of immunoglobulin K chain are all powerful secretory signal peptide and when the signal peptide of M-oncostatin or the signal peptide of immunoglobulin K- chain is added into endostatin, endostatin can be secreted outside the cells.

The present invention provides a virus specifically propagating in tumor cells and capable of expressing an angiogenesis inhibitor at high level and its nucleotide sequence encoding the angiogenesis inhibitor is the nucleotide sequence encoding the one containing angiostatin.

Angiostatin is the internal fragment of plasma plasminogen. Plasma plasminogen contains Kringle1-5 structure, wherein Kringle site is a structure of 3 disulfide-bonded structures in annulation. Each Kringle structure comprises about 80 amino acid residues. There is 50% identity of sequences among the Kringle structures. Angiostatin has strong effect of repressing the formation of blood vessels. Generally, angiostatin is meant the 38 kDa protein fragment of Plasma plasminogen containing the Kringle1-4 structure. Recently, there is study to compare the action of hydrolysis fragment Kringle1-4 of Plasma plasminogen against endothelial cells of blood vessels with that of Kringlel-3, showing that the action of Kringle1-3 is much stronger than that of Kringk1-4. The study on the action of recombinant Kringle1 Kringle2, Kringle3 and Kringle4 against endothelial cells of blood vessels showed that: the action against endothelial cells of blood vessels is in order Kringle1> Kringle3> Kringle2, while Kringle4 almost has no activity. The activity of Kringle5 against endothelial cells of blood vessels is similar to or stronger than that of the Kringle1-4.

The nucleotide sequence encoding angiostatin in the present invention is the nucleotide sequence encoding Kringle1-4 structure of plasma plasminogen.

The present invention provides a virus specifically propagating in tumor cells and capable of expressing an angiogenesis inhibitor at high level and the said nucleotide sequence encoding the angiogenesis inhibitor is the nucleotide sequences encoding Kringle1- structure of plasma plasminogen.

The present invention provides a virus specifically propagating in tumor cells and capable of expressing an angiogenesis inhibitor at high level and the said nucleotide sequence encoding the angiogenesis inhibitor is the nucleotide sequence encoding Kringle1-3 structure of plasma plasminogen.

The present invention provides a virus specifically propagating in tumor cells and capable of expressing an angiogenesis inhibitor at high level and its nucleotide sequence encoding the angiogenesis inhibitor is the nucleotide sequence encoding Kringle1-3 structure plus Kringle5 structure of plasma plasminogen.

The present invention provides a virus specifically propagating in tumor cells and capable of expressing an angiogenesis inhibitor at high level and its nucleotide sequence encoding the angiogenesis inhibitor is the nucleotide sequence encoding Kringle1 structure of plasma plasminogen.

The present invention provides a virus specifically propagating in tumor cells and capable of expressing an angiogenesis inhibitor at high level and its nucleotide sequence encoding the angiogenesis inhibitor is the nucleotide sequence encoding Kringle2 structure of plasma plasminogen.

The present invention provides a virus specifically propagating in tumor cells and capable of expressing an angiogenesis inhibitor at high level and its nucleotide sequence encoding the angiogenesis inhibitor is the nucleotide sequence encoding Kringle3 structure of plasma plasminogen.

The present invention provides a virus specifically propagating in tumor cells and capable of expressing an angiogenesis inhibitor at high level and its nucleotide sequence encoding the angiogenesis inhibitor is the nucleotide sequence encoding Kringle5 structure of plasma plasminogen.

Interferon is widely used for anti-virus therapy. There are researches demonstrating that interferon has the obvious effect of repressing tumors in *vivo.* Experiments showed: interferon represses the growth and metastasis of tumors by repressing the formation of the newborn blood vessels of tumors.

The present invention provides a virus specifically propagating in tumor cells and capable of expressing an angiogenesis inhibitor at high level and its nucleotide sequence encoding the angiogenesis inhibitor is the nucleotide sequence encoding interferon-α

The present invention provides a virus specifically propagating in tumor cells and capable of expressing an angiogenesis inhibitor at high level and its nucleotide sequence encoding the angiogenesis inhibitor is the nucleotide sequence encoding interferon-β.

The present invention provides a virus specifically propagating in tumor cells and capable of expressing an angiogenesis inhibitor at high level and its nucleotide sequence encoding the angiogenesis inhibitor is the nucleotide sequence encoding interferon-γ.

The present invention provides a virus specifically propagating in tumor cells and capable of expressing an angiogenesis inhibitor at high level and its nucleotide sequence encoding the angiogenesis inhibitor is the nucleotide sequence encoding thrombospondin I. Thrombospondin I is a glycoproteinbe constituted by three identical 180 kDa subunits and it contains at least two acting domains to repress the formation of newborn microvessels.

The present invention provides a virus specifically propagating in tumor cells and capable of expressing an angiogenesis inhibitor at high level and its nucleotide sequence encoding the angiogenesis inhibitor is the nucleotide sequence encoding platelet factor 4. Platelet factor 4 is a blood platelet α granule protein and its domain for repressing the formation of newborn microvessels locates at the C-terminus, the heparin-binding domain.

The present invention provides a virus specifically propagating in tumor cells and capable of expressing an angiogenesis inhibitor at high level and its nucleotide sequence encoding the angiogenesis inhibitor is the nucleotide sequence encoding plasminogen activator inhibitor (PAI).

The present invention provides a virus specifically propagating in tumor cells and capable of expressing an angiogenesis inhibitor at high level and its nucleotide sequence encoding the angiogenesis inhibitor is the nucleotide sequence encoding fibronectin. Fibronectin is the main component on the surface of many normal cells and the potential cell diffusion factor. Fibronectin repress the formation of newborn blood vessels *in vivo.*

The present invention provides a virus specifically propagating in tumor cells and capable of expressing an angiogenesis inhibitor at high level, wherein the nucleotide sequence encoding the angiogenesis inhibitor is controlled by promoters. The said promoter may be one of the following promoters: the promoter of Simian virus40 (conventionally abbreviated to SV40), the LTR promoter of Rous Sarcoma Virus (conventionally abbreviated to RSV), the IE promoter of human cytomegalovirus (conventionally abbreviated to HCMV) and the like.

In the present invention, the nucleotide sequence encoding angiogenesis inhibitor is inserted into the non-essential region of genome of the above-mentioned modified virus. The nucleotide sequences encoding angiogenesis inhibitors described in the present invention are endostatin genes containing the secretory signal peptide which is the signal peptide of M-oncostatin and the signal peptide of immunoglobulin K chain, angiostatin gene (Kringle 1-4 structure of plasma plasminogen), Kringle1-5 structure of plasma plasminogen, Kringlel-3 structure of plasma plasminogen, Kringle 1-3 structure of plasma plasminogen plus the Kringle5 structure, Kringlel structure of plasma plasminogen, Kringle2 structure of plasma plasminogen, Kringle3 structure of plasma plasminogen, Kringle5 structure of plasma plasminogen, interferon-a gene, interferon-β gene, interferon-γ gene, thrombospondin gene, platelet factor 4 gene, plasminogen activator inhibitor (PAI) and fibronectin gene. Along with the replication of virus in tumor cells, the copy numbers of the nucleotide sequence encoding the angiogenesis inhibitor are increased and the tumor cells are made express angiogenesis inhibitor at high level, thereby repressing the formation of the tumor blood vessel and repressing the formation, growth and metastasis of tumors. Meanwhile, this modified virus vector can be used for killing some particular target cells in some cell mixtures. By making the modified virus propagate selectively in this particular target cells, this particular target cells will be killed selectively by the propagated virus. By mixing the modified virus with cell compounds *in vitro* culture or in animal, the virus can propagate only in target cells, i.e., except the target cells, other cells can't be killed by this kind of virus. The virus propagates and replicates in the target cell, and thereby the target cells in mixed cells are killed. Once the target cells are destroyed, the virus can't propagate again.

In another aspect, the present invention provides a method of using the viruses described in the present invention for treating mammal tumors, especially human tumors. It includes the steps as follows: 1) *in vitro* or *in vivo* infecting tumor cells using the said virus, 2) making the virus selectively replicate and propagate substantially limited in tumor cells, leading to the increase of the copy numbers of the nucleotide sequence encoding the angiogenesis inhibitor and the expression amount of angiogenesis inhibitor in the tumor cells; whereby repressing the vascularization of tumors, specifically killing tumor cells directly to repress the formation, growth and metastasis of tumors. Mammals described in the present invention include, but are not limited to, humans, monkeys, cows, sheep, pigs, dogs, cats and the like.

In another aspect, the present invention further provides a method of using the viruses described in the present invention for treating mammal tumors, especially human tumors, wherein the method further include administration of the chemical antineoplastic drugs prior to, concurrently with and/or subsequent to the infection of viruses described in the present invention in tumor cells.

To further increase the therapeutic efficacy, viruses specifically propagating in tumor cells and capable of expressing an angiogenesis inhibitor at high level described in the present invention may be used for tumor therapy together with the conventional chemotherapy drugs (e.g., cis-platin, 5-fluorine uridine, mitocin-C and the like), biotoxins (e.g. snake toxin), monoclonal antibodies. It has the better effects for anti-tumor drugs. In another embodiment of the present invention, the virus of the present invention can be combined with X-ray and can produce better effects of anti-tumors.

The present invention provides a virus specifically propagating in tumor cells and capable of expressing an angiogenesis inhibitor at high level and the said virus can specifically repress the growth of tumor cells through the replication and propagation of virus itself in tumor cells.

The present invention provides a virus specifically propagating in tumor cells and capable of expressing an angiogenesis inhibitor at high level for *in vitro* infecting tumor cells. The virus replicates and propagates in the tumor cells, resulting in the copy number of the nucleotide sequence encoding angiogenesis inhibitor and the expression amount of angiogenesis inhibitor increased.

The present invention provides a virus specifically propagating in tumor cells and capable of expressing an angiogenesis inhibitor at high level. The virus can be used for selectively replicating and propagating in tumor cells, leading to the increases of the copy numbers of the nucleotide sequence encoding angiogenesis inhibitor and the expression amount of angiogenesis inhibitor in the tumor cells, thereby repressing the vascularization of tumors, repressing the formation, growth and metastasis of tumors. Meanwhile, the virus is able to propagate in and only limited in tumor cells, and also able to specifically kill tumor cells directly.

In still an aother aspect, the present invention. provides uses of the virus described in the present invention for repressing the growth of tumor cells.

Compared with current tumor therapies, the advantageous effects of the present invention are shown as follows:

The present invention provides a virus for the treatment of tumors specifically propagating in tumor cells and capable of expressing an angiogenesis inhibitor at high level. Animal experiments identified that the recombinant viruses may be used for treating tumors.

The present invention provides a method for constructing a virus specifically propagating in tumor cells and capable of expressing an angiogenesis inhibitor at high level. Generally, the said method is easily available and can be used for constructing viruses specifically propagating in tumor cells and capable of expressing an angiogenesis inhibitor at high level.

The present invention provides a virus capable of killing tumor cells both *in vivo and in vitro* without affecting normal cells, and furthermore, expressing angiogenesis inhibitor at high level in tumor cells both *in vivo and in vitro.* Combined with chemical anti-tumor drugs, it will kill tumor cells more efficiently, obtaining the objective of using the virus for tumor therapy with high efficiency and low toxicity.

Human adenovirus is classified into six different subgenuses: A, B, C, D, E and F. Their host cell tropism, neoplasia, as well as the nature of the disease induced by the virus are not identical. The present invention employs 5-type of adenoviral C subgenus (Ad5) as an exemplification to further describe the present invention in details. All the construction means of the present invention are available to those skilled in the art.

### Description of the Drawings

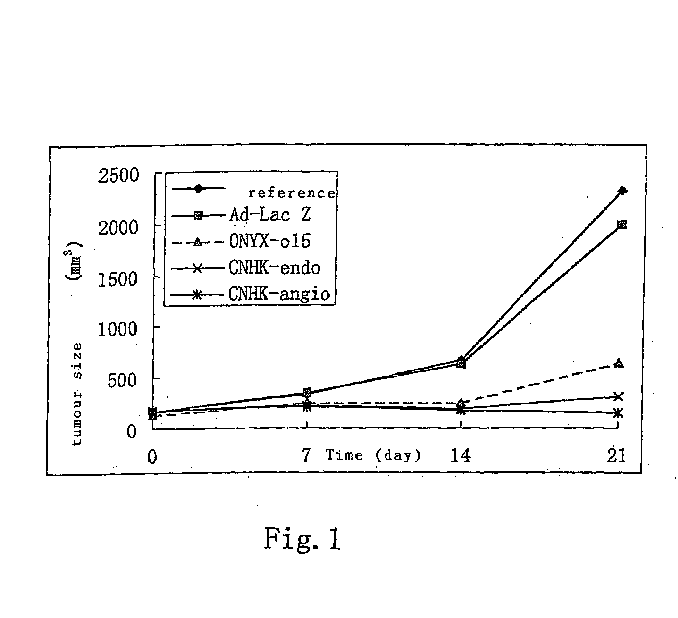

Figure 1 is the results of treating nude mice against transplanted human colon cancer *in vivo* using the recombinant adenovirus (CNHK-endostatinl-1, abbreviated to CNHK-endo) of the present invention with a partial deletion in E1b55kDa protein gene carrying endostatin gene and the recombinant adenovirus (CNHK-angiostatin1-1, abbreviated for CNHK-angio) of the present invention with a partial deletion in E1b55kDa protein gene carrying angiostatin gene, wherein the treatment times are indicated on the X-axis and tumor physical volumes are indicated on the Y-coordinate.

### Example

The molecular biology methods useful in the present invention, for example, the conventional methods such as restriction enzyme digestion, ligation, transformation, hybridization and the like, unless otherwise specified, are carried out with reference to Sambrook, *Molecular Cloning, A Laboratory Manual* (the second edition) and the instructions recommended by the manufacturers.

### Example 1: The construction of an adenovirus vector carrying a deletion of E1 region of human endostatin gene.

Vector pCA13 was commercially available from Microbix Biosystems Inc. (Toronto, Canadia). The pCA13 contains a 5-type adenovirus sequence from bp22 to 5790 and a deletion from 342bp to 3523bp in E1 region. IE promoter (-299--+72) of human cytomegalovirus (HCMV) and SV40 polyadenylation tail signal sequences were inserted into the deletion region of E1 Polymerase Chain Reaction (PCR) was used for amplifying human endostatin gene. The total RNA of human was extracted from the fresh and normal human liver tissue. Using RT- PCR (For procedures, see, the specification of SuperScrip^{TM} First―Strand Synthesis System for RT―PCR Kit, GIBCOBRL Company), the human endostatin gene was amplified using random primers. By two-step Polymerase Chain Reacting (PCR) (For procedures, see, White BA ed., *PCR Protocols Current Methods and Applications,* Humana Press Inc. 1993), an M-oncostatin signal peptide was added before the gene and EcoRI and XbaI restriction enzyme sites were introduced before and at the end of the gene.

Primer 1:GGG GAA TTC ACC ATG GGG GTA CTG CTC ACA CAG AGG

EcoRI

ACG CTG CTC AGT CTG GTC CTT GCA CTC (SEQ ID No: 1)

Primer 2:CTG CTC AGT CTG GTC CTT GCA CTC CTG TTT CCA AGC ATG GCG
AGC CAC CGC GAC TTC CAG (SEQ ID No: 2)

Primer 3:GCT CTA GAC TAT TAC TTG GAG GCA GTC ATG AAG CTG

XbaI

TTC TCA ATG CAT AGC ACG ATG TAG GCG TG (SEQ ID NO: 3)

Wherein, the first PCR amplication was carried out using RT-PCR products as a template, primer 2 and the primer 3 as primers, the PCR conditions are: the reverse transcription products 1µl, 10×buffer 5µl, 2 mmol/L dNTP 2µl, each primer 0.1µg, Pfu DNA polymerase 3U. Water was added up to the total volume 50µl, 94°C, 5 minutes for denaturation, then 94°C for 30 seconds, 50°C for 30 seconds, 72°C for one minute, totally 30 cycles, 72°C for 10 minutes for extension and the 615 bp fragments were recovered. Again the second PCR amplification was carried out using primer 1 and primer 3 as the primers and the 615 bp fragment as a template. The PCR conditions are: the products from the first amplification 1µl, 10×buffer 5µl, 2 mmol/L dNTP 2µl, each primer 0.1µg, Pfu DNA polymerase 3 U. Water was added up to the total volume 50µl, 94°C, 5 minutes for denaturation, then 94°C for 30 seconds, 50°C for 30 seconds, 72°C for one minute, totally 30 cycles, 72°C for 10 minutes for extension and the 647bp fragment was recovered. The recovered fragments were digested with EcoRI and Xbal and inserted into the vector pbluescript IDCS (+) (ATCC Company, USA) for sequencing, and the nucleotide sequence shown as SEQ ID NO: 4 was obtained, wherein the nucleotide sequence from bp 10 to 82 encodes the used M-oncostatin signal peptide and the nucleotide sequence from bp 83 to 632 encodes the said endostatin. The said fragments were from digestion with EcoRI and XbaI and inserted in orientation into the EcoRI-XbaI site of vector pCA13, designated pCA13-human endostatin.

### Example 2: The construction of an adenovirus vector with E1 deletion of an angiostatin gene.

Polymerase Chain Reacting (PCR) was performed for amplifying human angiostatin gene. Total RNA was extracted from the fresh and normal human liver tissues. By using RT-Polymerase Chain Reacting (PCR), the angiostatin gene was amplified using random primers. By using two-step Polymerase Chain Reacting (PCR) (For procedures, see, White BA ed., *supra),* both EcoRI and XbaI restriction enzyme sites were introduced in the end of the gene.

Primer 4: 5'-AGCGAATTCCAAAATGGAACATAAGG-3' (SEQ ID NO: 5)

EcoRI    plasminogen: 49-67

Primer 5: 5'-ACACTTTTCCTTGACCTGATTTCAG3'(SEQ ID NO:6)

plasminogen: 348-343+111-94

Primer6: 5'-CTGAAATCAGGTCAAGGAAAAGTGTATCTCTCAGAGTGC-3'
(SEQ ID NO: 7)
plasminogen: 94-111+343-363

Primer 7:5'-AGCCTCGAGCTATTACGCTTCTGTTCCTGAG-3'(SEQ ID NO: 8)

XhoI (2 stop)      plasminogen: 1431-1416

The first PCR was carried out using RT-PCR products and primers 4 and 5, primers 6 and 7, respectively, and then both PCR products were mixed together for the second PCR using primers 4 and 7. The 1168bp-fragment was recovered, digested with EcoRI and XhoI and then inserted into vector pbluescript IIKS (+) (ATCC Company, USA) for sequencing. The result is shown as SEQ ID NO: 9, wherein the nucleotide sequence from bp11 to 66 encodes the used signal peptide and the nucleotide sequence from bp 61 to 1155 encodes the said angiostatin. The said fragment was from digestion with EcoRI and Xbal and inserted in orientation into the EcoRI -Xbal of vector pCA13, designated pCA13-human angiostatin.

### Example 3: The construction of vector that contains a partial deletion of an adenovirus E1b 55Kda protein gene and a stop codon inserted in the deletion region

Vector pXC. 1 was commercially available from Microbix Biosystems Inc. (Toronto), Canadia. pXC.1 contains an adenovirus 5 sequence from bp22 to 5790. The said vector was digested at bp3329 with endonuclease BglII, then partially digested with endonuclease HindIII. A 9372-bp DNA fragment was recovered and filled in 3' with Klenow fragment of Escherichia coli DNA polymerase I. Thus the pXC. 1 vector with a deletion region from bp2809 to bp3329 was obtained.

Two DNA oligonucleotides were synthesized and made an adaptor. The nucleotide sequences of the DNA oligonucleotides are:

Primer 8 TAATGAGTAACTAA (SEQ ID No: 10)

Primer 9 TTAGTTACTCATTA (SEQ ID No: 11)

0.1µg of each DNA oligonucleotide fragment were mixed together, denatured at 100°C for 5 minutes, then renatured by slowly reducing temperature and phosphorylated using T4 bacteriophage polynucleotide kinase following renaturation. The phosphorylated adaptor was ligated with the pXC.1 vector with a deletion region from bp2809 to bp3329, designated pXC-del the E1b.

Primers near the two ends into which the adaptor was inserted were synthesized and they were, respectively,

Primer 10 CTG GCC AAT ACC AAC CTT A (SEQ ID NO: 12)

Primer 11 ATA TGA GCT CAC AAT GCT TC (SEQ ID NO: 13)

PCR was carried out using the above-mentioned pXC- de1 E1b as a template for in vitro amplification and the PCR product was sequenced. The result is shown as SEQ ID NO: 14. Wherein pXC-de1 E1b has a deletion region of 2809-3329 in vector pXC.1, the sequence TAAGAGTAACTAA was insert into the said region and the BglII digestion site was retained after two stop codons.

### Example 4: The constrution of an adenovirus vector carrying human endostatin or angiostatin and having a partial deletion in E1b 55 Kda gene

pCA13-human endostatin and pCA13-human angiostatin were digested with Bg1II, respectively. A 1237bp-fragment (containing IE promoter (-299--+72) of human cytomegalovirus (HCMV)), human endostatin gene containing M-oncostatin signal peptide and SV40 poly A tail signal) and a 1758bp-fragment (IE promoter (-299--+72) of human cytomegalovirus (HCMV), human angiostatin gene and SV40 poly A tail signal) were recovered, respectively. The recovered fragments were inserted into the Bgl II site of pXC-del Elb and PCR was used for identifying the sense or antisense orientation of insertion: the upstream primer of Bgl II: CTG GCC AAT ACC AAC CTT A (SEQ ID NO: 12) was used for amplification with 5' and 3'- primers of human endostatin and 5' and 3'-primers of human angiostatin, respectively.

### 1) PCR was carried out using this upstream primer and human endostatin 5'-primer and 3'-primer.

Primer 12, i.e., human endostatin 5'-primer (located at 110-131bp of M-oncostatin
signal peptide and human endostatin gene) TCC ACC TGG TTG CGC TCA ACA G
(SEQ ID NO: 15)

Primer 13, i.e., human endostatin 3'-primer (located at 577-600bp of M-oncostatin
signal peptide and human endostatin gene) AGC ACG ATG TAG GCG TGA TGG C
(SEQ ID NO: 16).

A 1056bp-fragment was obtained by amplification using primers 10 and 13, indicating that the PCR product contains the IE promoter (-299--+72) of human cytomegalovirus (HCMV), human endostatin gene containing the signal peptide of M-oncostatin and SV40 poly A tail signal were inserted into the BglII site of pXC-del Elb in sense orientation, designated pXC-del E1b-endostatin 1.

A 752bp-fragment was obtained by amplification using primers 10 and 12, indicating that the PCR product contains the IE promoter (-299--+72) of human cytomegalovirus (HCMV), human endostatin gene containing the signal peptide of M-oncostatin and SV40 poly A tail signal were inserted in antisense orientation into the BglII site of pXC-del Elb, designated pXC-del Elb-endostatin 2.

### 2 )PCR was carried out using this upstream primer and human angostatin 5'-primer and 3'-primer.

Primer 14, i.e., human angostatin 5'-primer (located at 11-32bp of human angostatin
gene) ATG GAA CAT AAG GAA GTG GTT C (SEQ ID NO: 17)

Primer 15, i.e., human angostatin 3'-primer (located at 823-842bp of human angostatin
gene) AGG AGT CAC AGG ACG GTA TC (SEQ ID NO: 18)

A 1298bp-fragment was obtained by amplification using primers 10 and 15, indicating that the PCR product contains IE promoter (-299--+72) of human cytomegalovirus (HCMV), human angostatin gene and SV40 poly A tail signal were inserted in sense orientation into the BglII site of pXC-de1 E1b, designated pXC-del Elb-angiostatin 1.

A 1374bp-fragment was obtained by amplification using primers 10 and 14, indicating that the PCR product contains IE promoter (-299--+72) of human cytomegalovirus (HCMV), human angostatin gene and SV40 poly A tail signal were inserted in antisense orientation into the BglII site of pXC-del Elb, designated pXC-del Elb-angiostatin 2.

### Example 5: The recombinant adenovirus carrying human endostatin or angiostatin and having a partial deletion in E1b55 Kda gene

The E1 transformed human embryonic kidney cell line, i.e., cell line 293 was commercially available from Microbix Biosystems Inc. (Toronto), Canadia and derived from a human embryonic kidney cell transformed by cleaved adenovirus 5-type DNA. The cell line contains and expresses the E1 region of adenovirus 5-type. Furthermore, it can be transformed by adenovirus DNA with a high transfection efficiency. 293 cells were co-transfected with one plasmid containing the left arm of adenovirus 5-type together with another plasmid containing the right arm of adenovirus 5-type, whereby adenoviruses with the ability for infection by homologous recombinant were obtained. Cell line 293 was co-transfected by those derived as example 1-4 pXC-del E1b-endostatin 1, pXC-del E1b-endostatin 2, pXC-del Elb-angiostatin 1 or pXC-del Elb-angiostatin 2 and plasmid pBHG10 containing the right arm of adenovirus 5-type or pBHGE3 by Lipofectamine. For detailed procedures, see, the manipulation instruction of GIBOCO BRL Company. pBHG10 and pBHGE3 were commercially available from Microbix Biosystems Inc. (Ontario), Canadia and pBHG10 contains the right arm of adenovirus 5-type but has a deletion of E3 region while PBHGE3 contains the right arm of adenovirus 5-type and E3 region. 9-14 days after co-transfection, virus plaques were obtained. By purification of the virus plaques for three times, the recombinant adenoviruses were obtained and designated CNHK-endostatin1-1, CNHK-endostatin1-2, CNHK-endostatin2-1, CNHK-endostatin2-2 and CNHK-angiostatin1-1, CNHK-angiostatin1-2, CNHK-angiostatin2-1 and CNHK-angiostatin2-2. For the detailed methods for cell transfection, see, Murray EJ ed., *Gene Transfer and Expression Protocols,* Humana Press, 1991, wherein for the methods for construction of a recombinant adenovirus, see, example 1-4. The genomic structure and specific name of each obtained virus are shown in table 1 below, respectively.

**Table 1**

| Virus | Name | Plasmid containing the left arm of Ad5 | Plasmid containing the right arm of Ad5 |
|---|---|---|---|
| Ad5-de1 E1b-endostatin 1 | CNHK-endostatin1-1 | pXC-de1 E1b-endostatin 1 | PBHGE3 |
| Ad5-de1 E1b-endostatin 2 | CNHK-endostatin1-2 | pXC-de1 E1b-endostatin 2 | PBHGE3 |
| Ad5-de1 E1b E3-endostatin 1 | CNHK-endostatin2-1 | pXC-de1 E1b- endostatin 1 | PBHG10 |
| Ad5-de1 E1b E3- endostatin 2 . | CNHK-endostatin2-2 | pXC-de1 E1b-endostatin 2 | PBHG10 |
| Ad5-de1 E1b- angiostatin 1 | CNHK-angiostatin1-1 | pXC-de1 E1b-angiostatin 1 | PBHGE3 |
| Ad5-de1 E1b-angiostatin 2 | CNHK-angiostatin1-2 | pXC-de1 E1b-angiostatin 2 | PBHGE3 |
| Ad5-de1 E1b E3- angiostatin 1 | CNHK- angiostatin2-1 | pXC-de1 E1b-angiostatin 1 | PBHG10 |
| Ad5-de1 E1b E3- angiostatin 2 | CNHK- angiostatin2-2 | pXC-de1 E1b-angiostatin 2 | PBHG10 |

Adenoviruses propagated in 293 cells on a large scale and can be purified massively by cesium chloride gradient centrifugation ( for detailed procedures, see, Murray EJ ed., *supra).*

Ad5-del E1b-endostatin 1(CNHK-Endostatin-1-1) is an adenovirus 5-type and has a deletion mutation region between 2809-3329bp (the partial sequence of E1b 55Kda gene). The sequence TAATGAGTAACTAA, which contains two stop codons, was inserted into the said deletion mutation region and the sequence containing IE promoter (-299--+72) of human cytomegalovirus (HCMV), human endostatin gene containing the signal peptide of M-oncostatin and SV40 poly A tail signal sequence were inserted into the BglII site in sense orientation after the stop codons. Other sequences of the virus are the same as those of Ad5.

Ad5-de1 E1b-endostatin2 (CNHK-Endostatin-1-2) is an adenovirus 5-type and has a deletion mutation region between 2809-3329bp (the partial sequence of Elb 55Kda gene). The sequence TAATGAGTAACTAA, which contains two stop codons, was inserted into the said deletion mutation region and the sequence containing IE promoter (-299--+72) of human cytomegalovirus (HCMV), human endostatin gene containing the signal peptide of M-oncostatin and SV40 poly A tail signal sequence were inserted into the Bgill site in antisense orientation after the stop codons. Other sequences of the virus are the same as those of Ad5.

Ad5-de1 E1b E3-endostatin1 (CNHK-endostatin-2-1) is an adenovirus 5-type and has a deletion mutation region between 2809-3329bp (the partial sequence of Elb 55Kda gene). The sequence TAATGAGTAACTAA, which contains two stop codons, was inserted into the said deletion mutation region and the sequence containing IE promoter (-299--+72) of human cytomegalovirus (HCMV), human endostatin gene containing the signal peptide of M-oncostatin and SV40 poly A tail signal sequence. were inserted into the BglII site in sense orientation after the stop codons, meanwhile concomitant with a deletion of 28133-30818bp (the partial sequence of E3 region). Other sequences of the virus are the same as those of Ad5.

Ad5-de1 E1b E3-endostatin2 (CNHK-endostatin-2-2) is an adenovirus 5-type and has a deletion mutation region between 2809-3329bp (the partial sequence of E1b 55Kda gene). The sequence TAATGAGTAACTAA, which contains two stop codons, was inserted into the said deletion mutation region and the sequence containing IE promoter (-299--+72) of human cytomegalovirus (HCMV), human endostatin gene containing the signal peptide of M-oncostatin and SV40 poly A tail signal sequence was inserted into the BglII site in antisense orientation after the stop codons, meanwhile concomitant with a deletion of 28133-30818bp (the partial sequence of E3 region). Other sequences of the virus are the same as those of Ad5.

Ad5-del Elb-angiostatin 1(CNHK-angiostatin-1-1) is an adenovirus 5-type and has a deletion mutation region between 2809-3329bp (the partial sequence of E1b 55Kda gene). The sequence TAATGAGTAACTAA, which contains two stop codons, was inserted into the said deletion mutation region and the sequence containing IE promoter (-299--+72) of human cytomegalovirus (HCMV), human angiostatin gene and SV40 poly A tail signal sequence were inserted into the BglII site in sense orientation after the stop codons. Other sequences of the virus are the same as those of Ad5.

Ad5-de1 E1b-angiostatin2 (CNHK-angiostatin-1-2) is an adenovirus 5-type and has a deletion mutation region between 2809-3329bp (the partial sequence of Elb 55Kda gene). The sequence TAATGAGTAACTAA, which contains two stop codons, was inserted into the said deletion mutation region and the sequence containing IE promoter (-299--+72) of human cytomegalovirus (HCMV), human angiostatin gene and SV40 poly A tail signal sequence were inserted into the Bgl II site in antisense orientation after the stop codons. Other sequences of the virus are the same as those of Ad5.

Ad5-de1 E1b E3-angiostatin1 (CNHK-angiostatin-2-1) is an adenovirus 5-type and has a deletion mutation region between 2809-3329bp (the partial sequence of Elb 55Kda gene). The sequence TAATGAGTAACTAA, which contains two stop codons, was inserted into the said deletion mutation region and the sequence containing IE promoter (-299--+72) of human cytomegalovirus (HCMV), human angiostatin gene and SV40 poly A tail signal sequence were inserted into the Bgl II site in sense orientation after the stop codons, meanwhile concomitant with a deletion of 28133-30818bp (the partial sequence of E3 region). Other sequences of the virus are the same as those of Ad5.

Ad5-de1 E1b E3-angiostatin2 (CNHK-angiostatin-2-2) is an adenovirus 5-type and has a deletion mutation region between 2809-3329bp (the partial sequence of Elb 55Kda gene). The sequence TAATGAGTAACTAA, which contains two stop codons, was inserted into the said deletion mutation region and the sequence containing IE promoter (-299--+72) of human cytomegalovirus (HCMV), human angiostatin gene and SV40 poly A tail signal sequence were inserted into the Bgl II site in antisense orientation after the stop codons, meanwhile concomitant with a deletion of 28133-30818bp (the partial sequence of E3 region). Other sequences of the virus are the same as those ofAd5.

### Example 6: In vitro replicating and propagating in tumor cells, high-efficiently expressing human endostatin or angiostatin and killing specifically tumor cells of the recombinant adenovirus carrying human endostatin or angiostatin and having a partial deletion of Elb 55Kda gene

A recombinant adenovirus constructed as example 5 CNHK-endostatin1-1, CNHK-angiostatin1-1 and ONYX-015 (presented by Professor Berk AJ of University of California, USA) were used for infecting the liver cancer cell lines HeP 3B (commercially available from ATCC, USA) and HeP GII (commercially available from ATCC, USA), pancreatic cancer cell line panc-1 (commercially available from ATCC, USA), colon cancer cell line HT29 (commercially available from ATCC, USA), stomach cancer cell line SGC7901, cervix cancer cell line Hela (commercially available from ATCC, USA), melanoma cell line A375 (commercially available from ATCC, USA), lung cancer cell line A549 (commercially available from ATCC, USA), mammary cancer cell line MBD-231 (commercially available from ATCC, USA), 293 and human normal fibroblast, respectively. Cells were inoculated in a 6-well plate with 2 × 10⁵ cells/well and infected with recombinant adenoviruses CNHKEBV-endostatin1-1, CNHKEBV-angiostatin1-1 with 4 × 10⁵ pfu, respectively. After 96 hours, the viral titre was assayed by using cell line 293 and the propagation multiples were calculated. (for detailed procedures, see, Murray EJ ed., *supra).* The results are shown in Table 2 below.

**Table 2**

| | CNHK- endostatin1-1 | CNHK-angiostatin1-1 | ONYX-015 |
|---|---|---|---|
| Hep3B | 2510 | 1530 | 1580 |
| Hep GII | 5010 | 316 | 2000 |
| Panc-1 | 5010 | 398 | 631 |
| HT29 | 1260 | 10 | 79.4 |
| SGC7901 | 794 | 65.33 | 25.1 |
| Hela | 6310 | 60.77 | 3980 |
| A375 | 25.1 | 197.5 | 199.5 |
| A549 | 794 | 1000 | 2510 |
| MBD-231 | 79.4 | 79.6 | 79 |
| 293 | 2000 | 493 | 1500 |
| Human normal fibroblast | 1.4 | 1.2 | 2.4 |

The liver cancer cell line Hep3B and human normal fibroblast were infected by the following recombinant adenoviruses, CNHK-endostatin1-1, CNHK-angiostatin1-1 and CNHK-angiostatin1-2 at 1 MOI, respectively and incubated at 37°C for 1 hour. 7 days after infection, cells were collected and virus DNA was extracted using QIAamp DNA Blood mini kit (QIAGEN Company, German). For procedures, see, the manipulation instruction of QIAGEN Company. The extracted DNA was digested with NheI and XhoI, run in 0.8% agarose gel, transferred onto the nylon membrane and hybridized with ³²P-labeled human Ad5 1178bp of the BstXI-XhoI fragment (located at nt 4611-5789 of adenovirus) for Southern blot, using pXC.1 as the control of virus copies. The copy numbers of CNHK-endostatin1-1, CNHK-endostatin1-2, CNHK-angiostatin1 and CNHK-angiostatin1-2 in each cell of liver cancer cell line Hep3B and human normal fibroblast are 2×10⁴, 3×10⁴, 1×10⁴, 1×10⁴ and <10, <10, <10, <10, respectively.

The above-mentioned DNAs extracted from CNHK-endostatinl-1 and CNHK-endostatin1-2 were digested with BglII, respectively, run in 1% agarose gel electrophoresis, transferred onto nylon membrane and hybridized with the ³²P-labeled cDNA fragment (obtained by digesting pCA13-human endostatin with both EcoRI and Xbal and recovering the 637bp-fragment) of human endostatin as a probe for Southern blot, using pCA13-human endostatin as the control of virus copies. The copy numbers of CNHK-endostatinl-1, CNHK-endostatin1-2 in each cell of liver cancer cell line Hep3B and human normal fibroblast are 2 × 10⁴,3 × 10⁴ and <10, <10, respectively.

The above-mentioned DNAs extracted from CNHK-angiostatin1-1 and CNHK-angiostatin1-2 were digested with BglII, respectively, run in 1% agarose gel electrophoresis, transferred onto nylon membrane and hybridized with the ³²P-labeled cDNA fragment (obtained by digesting pCA 1-3-human angiostatin with both EcoRI and XbaI and recovering the 1168bp-fragment) of human angiostatin as a probe for Southern blot, using pCA13-human angiostatin as the control of virus copies. The copy numbers of CNHK-angiostatin 1-1, CNHK-angiostatin 1-2 in each cell of liver cancer cell line Hep3B and human normal fibroblast are 1 × 10⁴, 1 × 10⁴ and <10, <10, respectively.

### Example7: The results of research on the recombinant adenovirus carrying human endostatin or angiostatin and having a partial deletion of Elb 55Kda gene in nude mice

The recombinant adenovirus carrying human endostatin or angiostatin and having a partial deletion of Elb 55Kda gene was used for treating transplanted tumors in nude mice. The research demonstrated that the said virus can express the human endostatin or angiostatin at high level, repress the formation of the nascent blood vessel of tumors, repress the formation, growth and metastasis of tumors. Meanwhile, the said virus is able to propagate in and only limited in tumor cells and also able to specifically kill tumor cells directly.

The 4-5-week-old nude mice were inoculated subcutaneously with colon cancer cell line HT29 with 1x10⁷ and two weeks later, treated with the propagative recombinant adenoviruses CNHK-endostatin1-1, CNHK-angiostatin1-1 and ONYX-015 with 1×10⁹ pfu or the control adenovirus Ad5-Lac Z with the same dose. The tumors of the untreated control group and the group treated with the control Ad5-Lac Z increased by more than 10 times 4 weeks later. On the contrary, the tumors of the treated group with CNHK-endostatin1-1 and CNHK-angiostatin1-1 do not increase, part of tumors shrank and the curative effect is better than that of ONYX-015. The results are shown in Figure1.

The 4-5-week-old SCID mice were inoculated subcutaneously with liver cancer cell line Hep 3B with 1 × 10⁷ and two weeks later, treated with the propagative recombinant adenoviruses CNHK-endostatin1-1, CNHK-angiostatin1-1 with 1×10⁹ pfu or the control adenovirus Ad5-Lac Z with the same dose. The tumors of the untreated control group and the group treated with the control Ad5-Lac Z increased by more than 4 times 4 weeks later. On the contrary, the tumors of the treated group shrank obviously, and part of tumors disappeared completely.

### Example 8: The construction for vectors containing cis-acting elements of EB virus

pCAT-Basic was used as the basic vector (commercially available from Promega Company) and the family of 30bp repeats fragment of Orip of EB (FR, located at 7337-8190 bp of EB virus) combined with the basic promoter of SV40 (mini-SV40) was inserted into the mutiple cloning site of the pCAT-Basic. PCR was used for amplifying the promoter of Orip of EB and the mini-SV40 promoter. The template for the former, EB virus DNA was extracted from lymphoma cell line B95-8. For the method of extracting virus DNA, see, the manipulation instruction of QIAamp Blood kit provided by QIAGEN Company. The template for the latter is pCAT-Control (commercially available from Promega Company). The primers for FR of Orip of EB virus are:

Primer 16: 5'-primer (containing HindIII and AgeI sites) GGG AAG CTT ACC GGT
GCA TGC AGG AAA AGG ACA AGC (SEQ ID NO: 19)

Primer 17: 3'-primer (containing partial sequence of mini-SV40 promoter) GAG ATG
CAG ATC AAT GGC ACC CCG GGG AAT ACC (SEQ ID NO: 20)

The primers for mini-SV40 promoter are:

Primer 18: 5'-primer (containing partial FR sequence of Orip) GTG CCA TTG ATC
TGC ATC TCA ATT AGT CAG (SEQ ID NO: 21)

Primer 19: 3'-primer (containing SalI and AgeI sites) GCT AAA GTC GAC ACC
GGT AAG CTT TTT GCA AAA GCC TAG (SEQ ID NO: 22)

Two-step overlapping PCR was used with the conditions: plasmid 0.001µg, 10×buffer 5µl, 2 mmol/L dNTP 2µl, each primer 0.1µg, pfu DNA polymerase 3U. Water was added up to the total volume 50*µl*, and 94°C, 5 minutes for denaturation, then 94°C for 30 seconds, 50°C for 30 seconds, 72 C for 1.5 minutes, totally 30 cycles, 72°C for 10 minutes for extension. The FR sequence of Orip of EB virus and the mini-SV40 promoter were fused to generate fusion promoter. The PCR fragment of fusion promoter was digested with HindIII and Sail and inserted into the HindIII-Sa1I site of pCAT-Basic vector. By DNA sequencing for the fragment, the sequence of fusion promoter was corresponded completely to the published sequence, designated CHEB-FRSV40.

pBluescript II KS (+) (commercially available from ATCC Company, USA) was used as the basic vector and the family of 30bp repeats fragment of Orip of EB (FR, located at 7337-8190 bp of EB virus) combined with the basic promoter of HSV-TK (mini-TK) was inserted into the mutiple cloning site of pbluescript II KS(+). PCR was used for amplifying the family of 30bp repeats fragment of Orip of EB and mini-SV40 promoter. The template for the former, EB virus DNA was extracted from lymphoma cell line B95-8. The method of extracting virus DNA, see, the manipulation instruction of QIAamp Blood kit provided by QIAGEN Company. The template for the latter is pTAL-Luc (commercially available from Clontech Company).

The primers for amplifying FR of Orip of EB virus are as follows:

Primer 20: 5'-primer (containing XhoI site) GGG CAT CTC GAG GCA TGC AGG
AAA AGG ACA AGC (SEQ ID NO: 23)

Primer 21: 3'-primer (containing partial mini-HSV-TK promoter sequence) AGT CGG
GGC GGC AAT GGC ACC CCG GGG AAT ACC (SEQ ID NO: 24)

The primers for amplifying mini-HSV-TK promoter are as follows:

Primer 22: 5'-primer (containing partial FR sequence of Orip) GGT GCC ATT GCC
GCC CCG ACT GCA TCT GC (SEQ ID NO: 25)

Primer 23: 3'-primer (containing XbaI site) TTT TCT AGA CTT CTG CTT CAT
CCC CGT G (SEQ ID NO: 26)

Two-step overlapping PCR *(supra)* was used for generating the fusion promoter by fusing the FR sequence of Orip of EB virus with mini-TK promoter. The PCR fragment of fusion promoter was digested with Xho I and Xba I and inserted into the Xho I-Xba I site of pbluescript II KS (+) vector (commercially available from ATCC Company, USA). The fragment was used for DNA sequencing and the fusion promoter sequence was corresponded completely to the published sequence, designated CHEB-FRTK.

### Example 9: The construction of the attenuated propagative adenovirus vector carrying endostatin or angiostatin with the expression of E1A and E1B under control of cis-acting elements of EB virus.

Vector pXC.1 was commercially available from Microbix Biosystems Inc. (Toronto, Canadia) and contains an adenovirus 5-type sequence from bp22 to 5790. A new and unique Age I site was introduced 12 bp to the E1A initiation codon at 552bp of the vector by site mutagenisis two-step PCR (for procedures, see, Example 1). The primers are, respectively:

Primer 24: 5'-primer (containing EcoRI site) TTC AAG AAT TCT CAT GTT TG
(SEQ ID NO: 27)

Primer 25: 3'-primer (inserting an A to generate an AgeI site) CAG TCA CCG GTG

TCG GAG C (SEQ ID NO: 28)

Primer 26: 5'-primer (inserting a T to generate an AgeI site) GCT CCG ACA CCG
GTG ACT GA (SEQ ID NO: 29)

Primer 27: 3'-primer (containing an XbaI site) TTC TCT AGA CAC AGG TGA TG
(SEO ID NO: 30)

The site mutagenesis two-step PCR was used and PCR product fragment was inserted into pGEM-T-easy vector (for procedures, see, manipulation instruction of Promega Company) for DNA sequencing, designated pGEM-T-E1a. The result of DNA sequencing showed that a base T was inserted at bp552 of plasmid pXC.1, whereby generating a new AgeI site, and the other sequence is the same as that of pXC.1.

Plasmid pGEM-T-E1A and pXC.1 were digested with EcoRI and Xbal, respectively, and the fragment digested from pGEM-T-E1A was inserted into the EcoRI-BamHI site of pXC.1; whereby a base T was inserted at the 552bp of pXC.1 to generate an Agel site 12bp to the E1A initiation codon. The resulted plasmid was designated pXC.1-Age I.

CHEB was digested with AgeI, respectively and the digested fragment was inserted into the Agel site of plasmid pXC-Age I, respectively. PCR amplification was carried out using primer 24 and 19, respectively, and a 2050bp-fragment was obtained, indicating that the family of 30bp repeats fragment of Orip of EB combined with mini-SV40 promoter was inserted in sense oritention into the Agel site of plasmid pXC-Age I, i.e., 12 bp upstream to the E1A initiation codon of adenovirus, designated pXC-FRSVE1A. To further verify that the family of 30bp repeats fragment of Orip of EB combined with mini-SV40 promoter was inserted in sense orientation into Agel site of plasmid pXC-Age I.

Vector pXC-FRSVE1A was digested with EcoRI and Xbal in the present research, and the 2823bp-fragment was recovered and inserted into the EcoRI-Xbal site of vector pBluescript II SK for sequencing. The result verified that the family of 30bp repeats fragment of Orip of EB combined with mini-SV40 promoter had been inserted in sense orientation into the AgeI site of plasmid pXC-Age I and the sequence is described as SEQIDNO:31.

Vector pXC.1 was commercially available from Microbix Biosystems Inc. (Toronto, Canadia), and contains an adenovirus 5-type sequence from bp22 to 5790. Mutiple restriction enzyme sites including BagII, BamHI, XhoI and Xbal were introduced between the transcriptional start site and the initiation codon of E1B at 1686bp of the vector by site mutagenisis two-step PCR (for procedures, *supra).* The primers are, respectively:

Primer 28: 5'-primer (containing HindIII and HpaI sites) TTT TGC AAG CTT GTT
AAC GCC TTT GTT TGC TGA (SEQ ID NO: 32)

Primer 29: 3'-primer (generating 4 restriction enzyme sites) CTC GAG GGA TCC
AGA TCT GCG CAT TAT ATA CCC TTT AAG (SEQ ID NO: 33)

Primer 30: 5'-primer (generating 4 restriction enzyme sites) AGA TCT GGA TCC
CTC GAG TGA TCT AGA GGG CTA ATC TTG GTT ACA TC (SEQ ID NO: 34)

Primer 31: 3'-primer (containing a KpaI site) CCA GAA AAT CCA GCA GGT AAC
(SEQ ID NO: 35)

The site mutagenesis two-step PCR was used and PCR product fragment was digested with HindIII and Kpnl and inserted into the HindIII-KpnI site of vector pUC19 (obtained from ATCC Company, USA), designated pUC-E1B. By DNA sequencing for the fragment, the result showed that BagII, BamHI, XhoI and XbaI restriction sites were inserted between the transcriptional start site and the initiation codon of E1B and the other sequence was the same as that of pXC.1.

CHEB-FRTK was digested with XhoI and XbaI, respectively. The digested fragmet was inserted into the XhoI-XbaI site of plasmid pUC-E1B, that is, the family of 30bp repeats fragment of Orip of EB combined with mini-TK promoter was inserted in sense orientation between the transcriptional start site and the initiation codon of E1B, designated pUC-E1B-FRTK. Then PCR amplification was performed using primer 28 and 23 and a 1159bp-fragment was obtained, indicating that the FR of Orip of EB virus combined with mini-TK promoter was inserted in sense orientation into the XhoI-XbaI site of plasmid pUC. The result of DNA sequencing is shown as SEQ ID NO: 36.

pCA13-human endostatin and pCA13-human angiostatin were digested with BglII, respectively. A 1237bp-fragment (containing IE promoter (-299--+72) of human cytomegalovirus (HCMV)), human endostatin gene containing M-oncostatin signal peptide and SV40 poly A tail signal) and a 1758bp-fragment (IE promoter (-299--+72) of human cytomegalovirus (HCMV), human angiostatin gene and SV40 poly A tail signal) were recovered, respectively. The recovered fragments were inserted into the BglII site of pUC-E1B-FRTK and PCR was used for identifying the sense or antisense orientation of insertion. The primer upstream BglII is:

Primer 32: GTT AAC GCC TTT GTT TGC TGA (SEQ ID NO: 37).

### 1) PCR was carried out using this upstream primer and human endostatin 5'-primer and 3'-primer, respectively.

Primer 12, i.e., human endostatin 5'-primer (located at 110-131bp of M-oncostatin
signal peptide and human endostatin gene) TCC ACC TGG TTG CGC TCA ACA G

(SEQ ID NO: 15)

Primer 13, i.e., human endostatin 3'-primer (located at 577-600bp of M-oncostatin
signal peptide and human endostatin gene) AGC ACG ATG TAG GCG TGA TGG C
(SEQ ID NO: 16)

The results are: a 1122bp-fragment was obtained by amplification using primers 32 and 13, indicating that IE promoter (-299--+72) of human cytomegalovirus (HCMV), human endostatin gene containing the signal peptide of M-oncostatin and SV40 poly A tail signal were inserted in sense orientation into the BglII site of pUC-E1B-FRTK, designated pUC-E1B-FRTK-endostatin 1.

A 818bp-fragment was obtained by amplification using primers 32 and 12, indicating that IE promoter (-299-+72) of human cytomegalovirus (HCMV), human endostatin gene containing the signal peptide of M-oncostatin and SV40 poly A tail signal were inserted in antisense orientation into the BglII site of pUC-E1B-FRTK, designated pUC-E1B-FRTK-endostatin 2.

### 2) PCR wase carried out using this upstream primer and human angostatin 5'-primer and 3'-primer, respectively.

Primer 14, i.e., human angostatin 5'-primer (located at 11-32bp of human angostatin
gene) ATG GAA CAT AAG GAA GTG GTT C (SEQ ID NO: 17)

Primer 15, i.e., human angostatin 3'-primer (located at 823-842bp of human angostatin
gene) AGG AGT CAC AGG ACG GTA TC (SEQ ID NO: 18)

The results are: a 1364bp-fragment was obtained by amplification using primers 32 and 15, indicating that IE promoter (-299--+72) of human cytomegalovirus (HCMV), human angostatin gene and SV40 poly A tail signal were inserted in sense orientation into the BglII site of pUC-E1B-FRTK, designated pUC-E1B-FRTK-angostatin 1.

A 1440bp-fragment was obtained by amplification using primers 32 and 14, indicating that IE promoter (-299--+72) of human cytomegalovirus (HCMV), human angostatin gene and SV40 poly A tail signal were inserted in antisense orientation into the BglII site of pUC-E1B-FRTK, designated pUC-E1B-FRTK-angostatin 2

The obtained pUC-E1B-FRTK-endostatin1, pUC-E1B-FRTK-endostatin2, pUC-E1B-FRTK-angostatin1 and pUC-E1B-FRTK-angostatin2 were digested with both Hpal and KpnI restriction enzymes, respectively. The digested fragments were recovered and inserted into the HpaI-KpnI site of pXC-FRSVE1A, respectively, designated pXC-FRSVE1A-FRSVE1B-endostatin1, pXC-FRSVElA-FRSVE1B-endostatin2 pXC-FRSVE1A-FRSVE1B-angostatinl and pXC-FRSVE1A-FRSVE1B-angostatin2, respectively.

### Example 10: The recombination of the attenuated propagative adenovirus vectors carrying human endostatin or angiostatin and having the expression of E1A and E1B controlled under the cis-acting elements of EB virus

Cell line 293 was commercially available from Microbix Biosystems Inc. (Toronto, Canadia), and derived from human embryonic kidney cell transformed by cleaved adenovirus 5-type DNA. The cell line contains and expresses the E1 region of adenovirus 5-type. Furthermore, it can be transformed by an adenovirus DNA with high transfection efficiency. 293 cells were co-transfected with a plasmid containing the left arm of adenovirus 5-type together with another plasmid containing the right arm of adenovirus 5-type, whereby generating adenoviruses with the ability for infection by homologous recombination. Cell line 293 was co-transfected with pXC-FRSVE1A-FRSVE1B-endostatinl, pXC-FRSVE1A-FRSVE1B-endostatin2, pXC-FRSVE1A-FRSVE1B-angostatin1, pXC-FRSVE1A-FRSVE1B-angostatin2 and plasmid PBHG10 containing the right arm of adenovirus 5-type by Lipofectamine. For the detailed procedures, see, the manipulation instruction of GIBOCO BRL Company. PBHG10 was commercially available from Microbix Biosystems Inc. (Ontario, Canadia), and contains the right arm of adenovirus 5-type but has a deletion of E3 region. 9-14 days after co-transfection, virus plaques were obtained. By purification of the. virus plaques for three times, an adenovirus was obtained with the expression of E1A and E1B controlled under the cis-acting elements Orip FR of EB virus combined with mini-SV40 promoter and Orip FR combined with mini-HSV-TK promoter, carrying human endostatin or angiostatin, designated EBV FRSVEIA-FRTKE1B-endostatinl, EBV FRSVEIA-FRTKE1B-endostatin2, EBV FRSVEIA-FRTKE1B-angiostatinl and EBV FRSVE1A-FRTKE1B-angiostatin2, respectively and recorded as CNHKEBV-endostatin1, CNHKEBV-endostatin2, CNHKEBV-angiostatinl and CNHKEBV-angiostatin2, respectively.

The recombinant viruses constructed by the above-mentioned methods are shown in Table 3.

**Table 3**

| Virus | Name | Plasmid containing the left arm of Ad5 | Plasmid containing the right arm of Ad5 |
|---|---|---|---|
| EBV FRSVEIA-FRTKE1B-endostatin1 | CNHKEBV-endostatin1 | pXC-FRSVE1A-FRTKElB-endostatin 1 | PBHG10 |
| EBV FRSVEIA-FRTKE1B-endostatin2 | CNHKEBV-endostatin2 | pXC-FRSVE1A-FRTKE1B-endostatin 2 | PBHG10 |
| EBV FRSVEIA-FRTKE1B-angiostatin1 | CNHKEBV-angiostatin1 | pXC-FRSVE1A-FRTKE1B-angiostatin 1 | PBHG10 |
| EBV FRSVE1A-FRTKE1B-angiostatin2 | CNHKEBV-angiostatin2 | pXC-FRSVE1A-FRTKE1B-angiostatin 2 | PBHG10 |

Adenoviruses propagated in 293 cells on a large scale and can be purified massively by cesium chloride gradient centrifugation. EBV FRSVEIA-FRTK E1B-endostati 1 (CNHKEBV-endostatin1) is an adenovirus 5-type, wherein the cis-acting elements of EB virus, i.e., the Orip FR combined with mini-SV40 promoter and Orip FR combined with mini-HSV-TK promoter were inserted between the transcriptional start site and the encoding initiation site of E1A and E1B. Furthermore, a BglII restriction site was introduced between the region after stop codon of E1A and the upstream region of Orip FR combined with mini-HSV-TK promoter, wherein IE promoter (-299--+72) of human cytomegalovirus (HCMV), human endostatin gene containing the signal peptide of M-oncostatin and SV40 poly A tail signal sequence were inserted in sense orientation into the BglII site, concomitant with the deletion of 28133-30818bp (a partial sequence of E3 region). Other sequences of the virus are the same as those of Ad5. EBV FRSVEIA-FRTK E1B-endostatin2 (CNHKEBV-endostatin 2) is an adenovirus 5-type, wherein the cis-acting elements of EB virus, i.e., the Orip FR combined with mini-SV40 promoter and Orip FR combined with mini-HSV-TK promoter were inserted between the transcriptional start site and the encoding initiation site of E1A and E1B. Furthermore, a BglII restriction site was introduced between the region after stop codon of E1A and the upstream region of Orip FR combined with mini-HSV-TK promoter, wherein IE promoter (-299--+72) of human cytomegalovirus (HCMV), human endostatin gene containing the signal peptide of M-oncostatin and SV40 poly A tail signal sequences were inserted in antiense oritention into the Bg1II site, concomitant with the deletion of 28133-30818bp (a partial sequence of E3 region). Other sequences of the virus are the same as those of Ad5.

EBV FRSVEIA-FRTK E1B-angiostatin1 (CNHKEBV-angiostatinl) is an adenovirus 5-type, wherein the cis-acting element of EB virus, i.e., the Orip FR combined with mini-SV40 promoter and Orip FR combined with mini-HSV-TK promoter was inserted between the transcriptional start site and the encoding initiation site of E1A and E1B. Furthermore, a BglII restriction site was introduced between the region after stop codon of E1A and the upstream region of Orip FR combined with mini-HSV-TK promoter, wherein IE promoter (-299--+72) of human cytomegalovirus (HCMV), human angiostatin gene and SV40 poly A tail signal sequence were inserted in sense orientation into the BglII site, concomitant with the deletion of 28133-30818bp (a partial sequence of E3 region). Other sequences of the virus are the same as those of Ad5. EBV FRSVEIA-FRTK E1B-angiostatin2 (CNHKEBV-angiostatin2) is an adenovirus 5-type, wherein the cis-acting elements of EB virus, i.e., the Orip FR combined with mini-SV40 promoter and Orip FR combined with mini-HSV-TK promoter were inserted between the transcriptional start site and the encoding initiation site of E1A and E1B. Furthermore, a BglII restriction site was introduced between the region after stop codon of E1A and the upstream region of Orip FR combined with mini-HSV-TK promoter, wherein IE promoter (-299--+72) of human cytomegalovirus (HCMV), human angiostatin gene and SV40 poly A tail signal sequence were inserted in antisense orientation into the BglII site, concomitant with the deletion of 28133-30818bp (a partial sequence of E3 region). Other sequences of the virus are the same as those of AdS.

### Example 11 Research on the attenuated propagative adenovirus carrying human endostatin or angiostatin and having the expression of E1A and E1B controlled under the cis-acting elements of EB virus in vitro

The research on the attenuated propagative adenovirus carrying human endostatin or angiostatin and having the expression of E1A and E1B controlled under the cis-acting elements of EB virus *in vitro* demonstrated that the said virus can propagate, replicate and express human endostatin or angiostatin at high level in tumor cells infected or latently infected by EB and specifically kill tumor cells.

Lymphoma cell line Jijoye, 293 and the human normal fibroblast infected by EB virus were infected with CNHKEBV-endostatin1, CNHKEBV-endostatin2, CNHKEBV-angiostatinl and CNHKEBV-angiostatin2, respectively. Cells were inoculated in a 6-well plate with 2 × 10⁵ cells/well and infected with recombinant adenoviruses CNHKBBV-endostatin1, CNHKEBV-endostatin1, CNHKEBV-angiostatinl, CNHKEBV-angiostatin2 and the wild-type adenovirus 5-type with 4 × 10⁵ pfu, respectively. After 48 hours, the viral titre was assayed by using cell line 293. The results are shown in Table 4.

**Table 4**

| | 293 | Jijoye | Human normal fibroblast |
|---|---|---|---|
| The wild-type Ad5 | 1 × 10⁵ | 7 × 10⁴ | 5 × 10⁴ |
| CNHKEBV-endostatin 1 | 1 × 10⁵ | 4 × 10⁵ | 3 × 10 |
| CNHKEBV-endostatin 2 | 1 × 10⁵ | 4 × 10⁵ | 4 × 10 |
| CNHKEBV-angiostatin 1 | 1 × 10⁵ | 2.5 × 10⁵ | 2 × 10 |
| CNHKEBV angiostatin 2 | 1 × 10⁵ | 2.5 × 10⁵ | 2 × 10² |

Lymphoma cell line Jijoye and the human normal fibroblast infected by infective EB virus were infected by recombinant adenoviruses CNHKEBV-endostatin1, CNHKEBV endostatin2, CNHKEBV-angiostatin1, CNHKEBV-angiostatin2 at 1 MOI, respectively and incubated at 37°C for 1 hour. 7 days after infection, cells were collected and virus DNA was extracted using QIAamp DNA Blood mini kit (QIAGEN Company, German). For procedures, see, the manipulation instruction of QIAGEN Company. The extracted DNA was digested with NheI and XhoI, run in 0.8% agarose gel electrophoresis, transferred onto the nylon membrane and hybridized with ³²P-labeled human Ad5 1178bp of the BstXI-XhoI fragment (located at nt 4611-5789 of the adenovirus) for Southern blot, using pXC.1 as the control of virus copy nembers (for procedures, see, *Molecular Clonig:* α *laboratory manual,* Science Press, 1992). The virus copy numbers of CNHKEBV-endostatin1, CNHKEBV-endostatin2, CNHKEBV-angiostatin1 and CNHKEBV-angiostatin2 in each cell of Jijoye and the normal human fibroblast were 5 × 10⁴, 5 × 10⁴, 4× 10⁴, 4 × 10⁴ and <10, <10, <10, <10, respectively. The above-mentioned DNA extracted from CNHKEBV-endostatin1, CNHKEBV-endostatin2 were digested with BglII, respectively, run in 1% agarose gel electrophoresis, transferred onto a nylon membrane and hybridized with the ³²P-labeled cDNA fragment (obtained by digesting pCA13-human endostatin with both EcoRI and XbaI and recovering the 637bp-fragment) of human endostatin as a probe for Southern blot. Using pCA13-human endostatin as the control of virus copies, the virus copies of CNHKEBV-endostatin1 and CNHKEBV-endostatin2 in each cell of Jijoye and the normal human fibroblast were 5x10⁴, 5x10⁴ and <10, <10, respectively.

The above-mentioned DNA extracted from CNHKEBV-angiostatin1 and CNHKEBV-angiostatin2 were digested with BglII, respectively, run in 1% agarose gel electrophoresis, transferred onto nylon membrane and hybridized with the ³²P-labeled cDNA fragment (obtained by digesting pCA13-human angiostatin with both EcoRI and XbaI and recovering the 1168bp-fragment) of human angiostatin as a probe for Southern blot. Using pCA13-human endostatin as the control of virus copies (for procedures, see, *Molecular Clonig: a laboratory manual,* Science Press, 1992), the virus copies of CNHKEBV-angiostatin1 and CNHKEBV-angiostatin2 in each cell of Jijoye and the normal human fibroblast were 4×10⁴, 4 ×10⁴ and <10, <10, respectively.

### Example 12: Research on the attenuated propagative adenovirus carrying human endostatin or angiostatin and having the expression of E1A and E1B controlled under the cis-acting elements of EB virus in SCID mice

The research on the attenuated propagative adenovirus carrying human endostatin or angiostatin and having the expression of E1A and E1B controlled under the cis-acting elements of EB virus in SCID mice was carried out to treat transplanted tumors of tumor cells infected or latently infected by EB virus.

The 4-5-week-old SCID mice were inoculated subcutaneously with lymphoma cell line Jijoye with 5×10⁷ infected by EB virus and two weeks later, treated with the propagative recombinant adenoviruses CNHKEBV-endostatin1 and CNHKEBV-angiostatin1 with 5×10⁸ pfu or the control adnovirus Ad5-Lac Z with the same dose. The tumors of the untreated control group and the group treated with the control Ad5-Lac Z increased by more than 3 times 4 weeks later. On the contrary, the tumors of the treated group shrank obviously, and some of them disappeared completely.

序列表

<110>  VIRGENE BIOTECHNOLOGY LIMITED

<120>  高效表达肿瘤血管生成抑制因子的肿瘤细胞内特异性增殖的病毒及其用途

<130>  iec010042pct

<160>  36

<170>  PatentIn version 3.1

<210>  1
<211>  63
<212>  DNA
<213>  Artificial

<400>  1
ggggaattca ccatgggggt actgctcaca cagaggacgc tgctcagtct ggtccttgca      60

ctc                                                                    63

<210>  2
<211>  60
<212>  DNA
<213>  Artificial

<400>  2
ctgctcagtc tggtccttgc actcctgttt ccaagcatgg cgagccaccg cgacttccag      60

<210>  3
<211>  65
<212>  DNA
<213>  Artificial

<400>  3
gctctagact attacttgga ggcagtcatg aagctgttct caatgcatag cacgatgtag      60

gcgtg                                                                  65

<210> 4
<211> 641
<212> DNA
<213> Artificial

<400> 4
gaattcacca tgggggtact gctcacacag aggacgctgc tcagtctggt ccttgcactc 60

ctgtttccaa gcatggcgag ccacagccac cgcgacttcc agccggtgct ccacctggtt 120

gcgctcaaca gccccctgtc aggcggcatg cggggcatcc gcggggccga cttccagtgc 180

ttccagcagg cgcgggccgt ggggctggcg ggcaccttcc gcgccttcct gtcctcgcgc 240

ctgcaggacc tgtacagcat cgtgcgccgt gccgaccgcg cagccgtgcc catcgtcaac 300

ctcaaggacg agctgctgtt tcccagctgg gaggctctgt tctcaggctc tgagggtccg 360

ctgaagcccg gggcacgcat cttctccttt aacggcaagg acgtcctgag gcaccccacc 420

tggccccaga agagcgtgtg gcatggctcg gaccccaacg ggcgcaggct gaccgagagc 480

tactgtgaga cgtggcggac ggaggctccc tcggccacgg gccaggcctc ctcgctgctg 540

gggggcaggc tcctggggca gagtgccgcg agctgccatc acgcctacat cgtgctatgc 600

attgagaaca gcttcatgac tgcctccaag taatagtcta g 641


<210> 5
<211> 26
<212> DNA
<213> Artificial

<400> 5
agcgaattcc aaaatggaac ataagg 26


<210> 6
<211> 25
<212> DNA
<213> Artificial

<400> 6
acacttttcc ttgacctgat ttcag 25

<210> 7
<211> 39
<212> DNA
<213> Artificial

<400> 7
ctgaaatcag gtcaaggaaa agtgtatctc tcagagtgc 39

<210> 8
<211> 31
<212> DNA
<213> Artificial

<400> 8
agcctcgagc tattacgctt ctgttcctga g 31

<210> 9
<211> 1168
<212> DNA
<213> Artificial

<400> 9
gaattccaaa atggaacata aggaagtggt tcttctactt cttttatttc tgaaatcagg 60

tcaaggaaaa gtgtatctct cagagtgcaa gactgggaat ggaaagaact acagagggac 120

gatgtccaaa acaaaaaatg gcatcacctg tcaaaaatgg agttccactt ctccccacag 180

acctagattc tcacctgcta cacacccctc agagggactg gaggagaact actgcaggaa 240

tccagacaac gatccgcagg ggccctggtg ctatactact gatccagaaa agagatatga 300

ctactgcgac attcttgagt gtgaagagga atgtatgcat tgcagtggag aaaactatga 360

cggcaaaatt tccaagacca tgtctggact ggaatgccag gcctgggact ctcagagccc 420

acacgctcat ggatacattc cttccaaatt tccaaacaag aacctgaaga agaattactg 480

tcgtaacccc gatagggagc tgcggccttg gtgtttcacc accgacccca acaagcgctg 540

ggaactttgc gacatccccc gctgcacaac acctccacca tcttctggtc ccacctacca 600

gtgtctgaag ggaacaggtg aaaactatcg cgggaatgtg gctgttaccg tttccgggca 660

cacctgtcag cactggagtg cacagacccc tcacacacat aacaggacac cagaaaactt 720

cccctgcaaa aatttggatg aaaactactg ccgcaatcct gacggaaaaa gggccccatg 780

gtgccataca accaacagcc aagtgcggtg ggagtactgt aagataccgt cctgtgactc 840

ctccccagta tccacggaac aattggctcc cacagcacca cctgagctaa cccctgtggt 900

ccaggactgc taccatggtg atggacagag ctaccgaggc acatcctcca ccaccaccac 960

aggaaagaag tgtcagtctt ggtcatctat gacaccacac cggcaccaga agaccccaga 1020

aaactaccca aatgctggcc tgacaatgaa ctactgcagg aatccagatg ccgataaagg 1080

cccctggtgt tttaccacag accccagcgt caggtgggag tactgcaacc tgaaaaaatg 1140

ctcaggaaca gaagcgtaat agctcgag 1168

<210> 10
<211> 14
<212> DNA
<213> Artificial

<400> 10
taatgagtaa ctaa 14

<210> 11
<211> 14
<212> DNA
<213> Artificial

<400> 11
ttagttactc atta 14

<210> 12

<211> 19
<212> DNA
<213> Artificial

<400> 12
ctggccaata ccaacctta 19

<210> 13
<211> 20
<212> DNA
<213> Artificial

<400> 13
atatgagctc acaatgcttc 20

<210> 14
<211> 372
<212> DNA
<213> Artificial

<400> 14
ctggccaata ccaaccttat cctacacggt gtaagcttaa tgagtaacta agatctggaa 60

ggtgctgagg tacgatgaga cccgcaccag gtgcagaccc tgcgagtgtg gcggtaaact 120

attaggaacc agcctgtgat gctggatgtg accgaggagc tgaggcccga tcacttggtg 180

ctggcctgca cccgcgctga gtttggctct agcgatgaag atacagattg aggtactgaa 240

atgtgtgggc gtggcttaag ggtgggaaag aatatataag gtgggggtct tatgtagttt 300

tgtatctgtt ttgcagcagc cgccgccgcc atgagcacca actcgtttga tggaagcatt 360

gtgagctcat at 372

<210> 15
<211> 22
<212> DNA
<213> Artificial

<400> 15

tccacctggt tgcgctcaac ag 22

<210> 16
<211> 22
<212> DNA
<213> Artificial

<400> 16
agcacgatgt aggcgtgatg gc 22

<210> 17
<211> 22
<212> DNA
<213> Artificial

<400> 17
atggaacata aggaagtggt tc 22

<210> 18
<211> 20
<212> DNA
<213> Artificial

<400> 18
aggagtcaca ggacggtatc 20

<210> 19
<211> 36
<212> DNA
<213> Artificial

<400> 19
gggaagctta ccggtgcatg caggaaaagg acaagc 36

<210> 20
<211> 33
<212> DNA
<213> Artificial

<400> 20
gagatgcaga tcaatggcac cccggggaat acc 33

<210> 21
<211> 30
<212> DNA
<213> Artificial

<400> 21
gtgccattga tctgcatctc aattagtcag 30

<210> 22
<211> 39
<212> DNA
<213> Artificial

<400> 22
gctaaagtcg acaccggtaa gctttttgca aaagcctag 39

<210> 23
<211> 33
<212> DNA
<213> Artificial

<400> 23
gggcatctcg aggcatgcag gaaaaggaca agc 33

<210> 24
<211> 33
<212> DNA
<213> Artificial

<400> 24
agtcggggcg gcaatggcac cccggggaat acc 33

<210> 25
<211> 29
<212> DNA
<213> Artificial

<400> 25
ggtgccattg ccgcccccgac tgcatctgc 29

<210> 26
<211> 28
<212> DNA
<213> Artificial

<400> 26
ttttctagac ttctgcttca tccccgtg 28

<210> 27
<211> 20
<212> DNA
<213> Artificial

<400> 27
ttcaagaatt ctcatgtttg 20

<210> 28
<211> 19
<212> DNA
<213> Artificial

<400> 28
cagtcaccgg tgtcggagc 19

<210> 29
<211> 20
<212> DNA
<213> Artificial

<400> 29
gctccgacac cggtgactga 20

<210> 30
<211> 20
<212> DNA

<213>  Artificial

<400>  30
ttctctagac acaggtgatg                                                    20


<210>  31
<211>  2823
<212>  DNA
<213>  Artificial

<400>  31
gaattctcat gtttgacagc ttatcatcga taagctttaa tgcggtagtt tatcacagtt      60

aaattgctaa cgcagtcagg caccgtgtat gaaatctaac aatgcgctca tcgtcatcct     120

cggcaccgtc accctggatg ctgtaggcat aggcttggtt atgccggtac tgccgggcct     180

cttgcgggat atcgtccatt ccgacagcat cgccagtcac tatggcgtgc tgctagcgct     240

atatgcgttg atgcaatttc tatgcgcacc cgttctcgga gcactgtccg accgctttgg     300

ccgccgccca gtcctgctcg cttcgctact tggagccact atcgactacg cgatcatggc     360

gaccacaccc gtcctgtgga tccgggcccc catttcccct cccttccagc tctctgcccc     420

ttttggattg aagccaatat gataatgagg gggtggagtt tgtgacgtgg cgcggggcgt     480

gggaacgggg cgggtgacgt agtagtgtgg cggaagtgtg atgttgcaag tgtggcggaa     540

cacatgtaag cgacggatgt ggcaaaagtg acgtttttgg tgtgcgccgg tgtacacagg     600

aagtgacaat tttcgcgcgg ttttaggcgg atgttgtagt aaatttgggc gtaaccgagt     660

aagatttggc cattttcgcg ggaaaactga ataagaggaa gtgaaatctg aataattttg     720

tgttactcat agcgcgtaat atttgtctag ggccgcgggg actttgaccg tttacgtgga     780

gactcgccca ggtgtttttc tcaggtgttt tccgcgttcc gggtcaaagt tggcgtttta     840

ttattatagt cagctgacgt gtagtgtatt tatacccggt gagttcctca agaggccact     900

cttgagtgcc agcgagtaga gttttctcct ccgagccgct ccgacaccgg tgcatgcagg     960

aaaaggacaa gcagcgaaaa ttcacgcccc cttgggaggt ggcggcatat gcaaaggata 1020

gcactcccac tctactactg ggtatcatat gctgactgta tatgcatgag gatagcatat 1080

gctacccgga tacagattag gatagcatat actacccaga tatagattag gatagcatat 1140

gctacccaga tatagattag gatagcctat gctacccaga tataaattag gatagcatat 1200

actacccaga tatagattag gatagcatat gctacccaga tatagattag gatagcctat 1260

gctacccaga tatagattag gatagcatat gctacccaga tatagattag gatagcatat 1320

gctatccaga tatttgggta gtatatgcta cccagatata aattaggata gcatatacta 1380

ccctaatctc tattaggata gcatatgcta cccggataca gattaggata gcatatacta 1440

cccagatata gattaggata gcatatgcta cccagatata gattaggata gcctatgcta 1500

cccagatata aattaggata gcatatacta cccagatata gattaggata gcatatgcta 1560

cccagatata gattaggata gcctatgcta cccagatata gattaggata gcatatgcta 1620

tccagatatt tgggtagtat atgctaccca tggcaacatt agcccaccgt gctctcagcg 1680

acctcgtgaa tatgaggacc aacaaccctg tgcttggcgc tcaggcgcaa gtgtgtgtaa 1740

tttgtcctcc agatcgcagc aatcgcgccc ctatcttggc ccgcccacct acttatgcag 1800

gtattccccg gggtgccatt gatctgcatc tcaattagtc agcaaccata gtcccgcccc 1860

taactccgcc catcccgccc ctaactccgc ccagttccgc ccattctccg ccccatggct 1920

gactaatttt ttttatttat gcagaggccg aggccgcctc ggcctctgag ctattccaga 1980

agtagtgagg aggctttttt ggaggcctag gcttttgcaa aaagcttacc ggtgactgaa 2040

aatgagacat attatctgcc acggaggtgt tattaccgaa gaaatggccg ccagtctttt 2100

ggaccagctg atcgaagagg tactggctga taatcttcca cctcctagcc attttgaacc 2160

acctaccctt cacgaactgt atgatttaga cgtgacggcc cccgaagatc ccaacgagga 2220

ggcggtttcg cagatttttc ccgactctgt aatgttggcg gtgcaggaag ggattgactt 2280

actcactttt ccgccggcgc ccggttctcc ggagccgcct cacctttccc ggcagcccga 2340

gcagccggag cagagagcct tgggtccggt ttctatgcca aaccttgtac cggaggtgat 2400

cgatcttacc tgccacgagg ctggctttcc acccagtgac gacgaggatg aagagggtga 2460

ggagtttgtg ttagattatg tggagcaccc cgggcacggt tgcaggtctt gtcattatca 2520

ccggaggaat acgggggacc cagatattat gtgttcgctt tgctatatga ggacctgtgg 2580

catgtttgtc tacagtaagt gaaaattatg ggcagtgggt gatagagtgg tgggtttggt 2640

gtggtaattt tttttttaat ttttacagtt ttgtggttta aagaattttg tattgtgatt 2700

tttttaaaag gtcctgtgtc tgaacctgag cctgagcccg agccagaacc ggagcctgca 2760

agacctaccc gccgtcctaa aatggcgcct gctatcctga gacgcccgac atcacctgtg 2820

tct 2823

<210> 32
<211> 33
<212> DNA
<213> Artificial

<400> 32
ttttgcaagc ttgttaacgc ctttgtttgc tga 33

<210> 33
<211> 39
<212> DNA
<213> Artificial

<400> 33
ctcgagggat ccagatctgc gcattatata ccctttaag 39

<210> 34
<211> 47
<212> DNA
<213> Artificial

<400> 34
agatctggat ccctcgagtg atctagaggg ctaatcttgg ttacatc 47

<210> 35
<211> 21
<212> DNA
<213> Artificial

<400> 35
ccagaaaatc cagcaggtaa c 21

<210> 36
<211> 1526
<212> DNA
<213> Artificial

<400> 36
gttaacgcct ttgtttgctg aatgagttga tgtaagttta ataaagggtg agataatgtt 60

taacttgcat ggcgtgttaa atggggcggg gcttaaaggg tatataatgc gcagatctgg 120

atccctcgag gcatgcagga aaaggacaag cagcgaaaat tcacgccccc ttgggaggtg 180

gcggcatatg caaaggatag cactcccact ctactactgg gtatcatatg ctgactgtat 240

atgcatgagg atagcatatg ctacccggat acagattagg atagcatata ctacccagat 300

atagattagg atagcatatg ctacccagat atagattagg atagcctatg ctacccagat 360

ataaattagg atagcatata ctacccagat atagattagg atagcatatg ctacccagat 420

atagattagg atagcctatg ctacccagat atagattagg atagcatatg ctacccagat 480

atagattagg atagcatatg ctatccagat atttgggtag tatatgctac ccagatataa 540

attaggatag catatactac cctaatctct attaggatag catatgctac ccggatacag 600

attaggatag catatactac ccagatatag attaggatag catatgctac ccagatatag 660

attaggatag cctatgctac ccagatataa attaggatag catatactac ccagatatag 720

attaggatag catatgctac ccagatatag attaggatag cctatgctac ccagatatag 780

attaggatag catatgctat ccagatattt gggtagtata tgctacccat ggcaacatta 840

gcccaccgtg ctctcagcga cctcgtgaat atgaggacca acaaccctgt gcttggcgct 900

caggcgcaag tgtgtgtaat ttgtcctcca gatcgcagca atcgcgcccc tatcttggcc 960

cgcccaccta cttatgcagg tattccccgg ggtgccattg ccgccccgac tgcatctgcg 1020

tgttcgaatt cgccaatgac aagacgctgg gcggggtttg tgtcatcata gaactaaaga 1080

catgcaaata tatttcttcc ggggacaccg ccagcaaacg cgagcaacgg gccacgggga 1140

tgaagcagaa gtctagaggg ctaatcttgg ttacatctga cctcatggag gcttgggagt 1200

gtttggaaga tttttctgct gtgcgtaact tgctggaaca gagctctaac agtacctctt 1260

ggttttggag gtttctgtgg ggctcatccc aggcaaagtt agtctgcaga attaaggagg 1320

attacaagtg ggaatttgaa gagcttttga aatcctgtgg tgagctgttt gattctttga 1380

atctgggtca ccaggcgctt ttccaagaga aggtcatcaa gactttggat ttttccacac 1440

cggggcgcgc tgcggctgct gttgcttttt tgagttttat aaaggataaa tggagcgaag 1500

aaacccatct gagcgggggg tacctg 1526

## Claims

1. A viruse specifically propagating in tumor cells and capable of expressing an angiogenesis inhibitor at high level, **characterized in that** the non-essential region of genome of the said virus comprises a nucleotide sequence encoding the angiogenesis inhibitor, wherein the said virus is selected from the group consisting of:
1) a wild-type virus specifically propagating in tumor cells;
2) a recombinant virus containing the cis-acting elements specifically activated in tumor cells between the transcriptional start site and the encoding start site in essential genes for propagation of an virus;
3) a recombinant virus comprising at least one loss of protein function in essential region for propagation and capable of specifically propagating in tumor cells.

2. A virus according to claim 1, **characterized in that** it comprises at least one cis-acting element specifically activated in tumor cells between the transcriptional start site and the encoding start site in essential region for propagation of virus, wherein the said cis-acting element is selected from the group consisting of: the enhancer and promoter of α -fetal protein, the enhancer and promoter of carcinoembryonic antigen, the enhancer and promoter of tyrosinase, the enhancer and promoter of ErbB2, the enhancer and promoter of ErbB3, the enhancer and promoter of ErbB4, the enhancer of DF3 mammary cancer-related antigen, the enhancer and promoter of prostaglandin-specific antigen, the enhancer and promoter of glandular kallikrein, Orip in EB virus, FR enhancer in Orip of EB virus and BamHI C-promoter of EB virus, Orip in EB virus combined with BamHI C-promoter of EB virus, FR enhancer in Orip of EB virus combined with the basic promoter of thymidine kinase of herpes simplex virus or the basic promoter of SV40 and the cis-acting elements specifically activated in the cells infected or latently infected by EB virus.

3. A virus according to claim 2, **characterized in that** the said virus is an adenovirus, and the essential genes for propagation of the adenovirus are one of the following early expression genes of an adenovirus: E1A, E1B, E2 or E4.

4. A virus according to claim 1, **characterized in that** the said virus is a recombinant adenovirus, wherein there is a loss of function in the protein encoded by E1B55Kda gene, E1B19Kda gene and/or E1A gene of the adenovirus.

5. A virus according to claim 1, **characterized in that** the said virus is a recombinant herpes simplex virus, wherein there is a loss of function in the protein encoded by ICP6 gene and/or double-copied ICP34.5 gene of the herpes simplex virus.

6. A virus according to claim 1, **characterized in that** the said nucleotide sequence encoding the angiogenesis inhibitor is selected from the group consisting of the nucleotide sequence encoding: endostatin, angiostatin, Kringle1-5 structure in plasma plasminogen, Kringle1-3 structure in plasma plasminogen, Kringle1-3 structure in plasma plasminogen plus Kringle5 structure, Kringlel structure in plasma plasminogen, Kringle2 structure in plasma plasminogen, Kringle3 structure in plasma plasminogen, Kringle5 structure in plasma plasminogen, interferon-α, interferon-β, interferon-γ, thrombospondin I, platelet factor 4, plasminogen activator inhibitor and fibronectin.

7. A virus according to claim 6, wherein the said nucleotide sequence encoding angiostatic suppressor further comprises a nucleotide sequence coding for a secretory signal peptide, and the said secretory signal peptide is selected from the signal peptide of angiogenesis inhibitor itself, the signal peptide of M-oncostatin and the signal peptide of immunoglobulin K chain.

8. A virus according to claim 6, wherein the expression of nucleotide sequence encoding the said angiogenesis inhibitor is further controlled by a promoter, and the said promoter is selected from the group consisting of SV40 promoter, RSVLTR promoter and/or IE promoter of human cytomegalovirus.

9. A method for treating mammal especially human tumors using the said virus according to claim 1, it includes the steps as follows: 1) *in vitro* or *in vivo* infecting tumor cells using the said virus, 2) making the virus selectively replicate and propagate substantially limited in tumor cells, leading to the increase of the copy numbers of the nucleotide sequence encoding the angiogenesis inhibitor and the expression amount of angiogenesis inhibitor in the tumor cells, whereby repressing the vascularization of tumors, specifically killing tumor cells directly to repress the formation, growth and metastasis of tumors.

10. A method according to claim 9, wherein it further comprises administration of the chemical antineoplastic drugs prior to, concurrently with and/or subsequent to the infection of tumor cells with the said virus according to claim 1.

11. Use of the virus according to claim 1 for suppressing the growth of tumor cells.
